# EUROPEAN PATENT APPLICATION

(11) **EP 4 239 077 A2**
(43) Date of publication of application: **06.09.2023**
(21) Application number: 23169604.8
(22) Date of filing: 19.03.2021
(51) Int. Cl.: C12Q 1/28, G01N 33/569, G01N 33/68

(54) **TRIAGING METHOD USING CELL FREE NUCLEOSOME LEVELS**

(30) Priority: 20.03.2020 GB 202004100; 07.04.2020 CN 202010265531; 06.05.2020 GB 202006723; 07.07.2020 GB 202010446; 10.09.2020 GB 202014263; 16.10.2020 GB 202016403; 30.11.2020 GB 202018835; 20.01.2021 GB 202100769
(62) Divisional of application: 21714838.6
(71) Applicant: Belgian Volition SRL, 5032 Isnes (BE)
(72) Inventor: MICALLEF, Jacob Vincent, BE-5032 Isnes (BE); ECCLESTON, Mark Edward, BE-5032 Isnes (BE); TERRELL, Jason Bradley, BE-5032 Isnes (BE)
(74) Representative: Sagittarius IP

(57) **Abstract**

The invention relates to using cell free nucleosome levels to identify patients at risk of developing a NETosis associated adverse reaction to the infection. The methods are used to monitor the progress of a disease and assigning a risk of an adverse outcome in a patient suffering from an infection.

## Description

### FIELD OF THE INVENTION

The present invention relates to the use of cell free nucleosomes as biomarkers in body fluid samples for patients with an infection, particularly for identifying patients at high risk of developing a NETosis associated adverse reaction to the infection. It also relates to the use of anti-nucleosome antibodies as therapeutic antibodies for the treatment of NETosis associated conditions.

### BACKGROUND OF THE INVENTION

Influenza spreads around the world in yearly outbreaks, resulting in about three to five million cases of severe illness and about 290,000 to 650,000 deaths. More recently, the emergence and rapid progression of a new infection, COVID-19, has escalated to pandemic status. Some infections lead to acute respiratory syndrome (ARS), acute respiratory distress syndrome (ARDS), or to severe acute respiratory syndrome (SARS) which are potentially lethal disease progressions requiring medical treatment. Infection outbreaks and pandemics place severe strain on international health care services, therefore methods of triaging patients to identify those who are most likely to require hospital intervention are critical in helping health care providers to prioritise patients, save lives and manage higher demands on medical services more effectively.

COVID-19, influenza and other infections have the potential to progress to the involvement of NETosis related complications which may be severe and can be lethal. Such complications include sepsis, a life-threatening organ dysfunction that can occur as a complication to infection. Methods to treat inappropriate NETosis, to identify individuals at high risk of NETosis related complications, to monitor the progress of such complications in need of such treatment, to monitor the efficacy of treatments and to monitor the progress of such disease are currently lacking.

Holdenrieder et al., Int. J. Cancer (2001) 95: 114-120 previously described detecting the level of nucleosomes in serum samples of patients with benign and malignant diseases. The epigenetic composition of circulating cell free nucleosomes in terms of their histone modification, histone variant, DNA modification and adduct content have also been investigated as blood based biomarkers in cancer, see WO 2005/019826, WO 2013/030577, WO 2013/030579 and WO 2013/084002.

There remains a need in the art to provide effective treatments for NETosis related conditions as well as for simple, cost-effective methods to identify and prioritize individuals likely to develop NETosis related complications with poor prognosis upon infection and to monitor treatment and progress of disease.

### BRIEF DESCRIPTION OF FIGURES

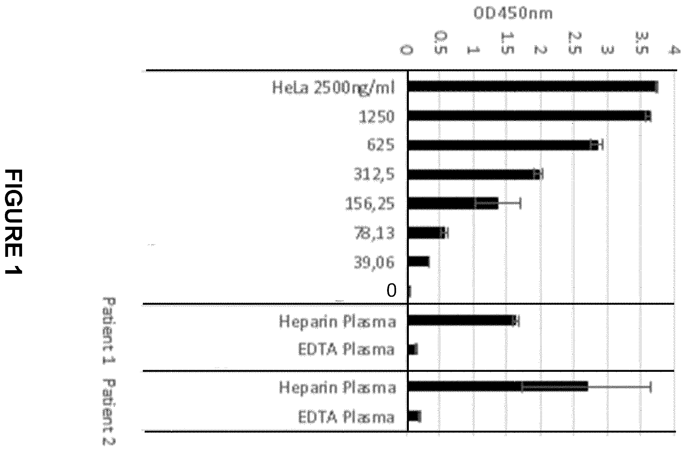

**Figure 1****.** The results of an immunoassay for neutrophil extracellular trap (NET) derived nucleosomes in EDTA plasma and heparin plasma samples taken from 2 healthy volunteers. The EDTA samples contain low levels of NET derived nucleosome material. In contrast, heparin induces NET formation and the heparin plasma samples contain high levels of induced NET derived nucleosomes.

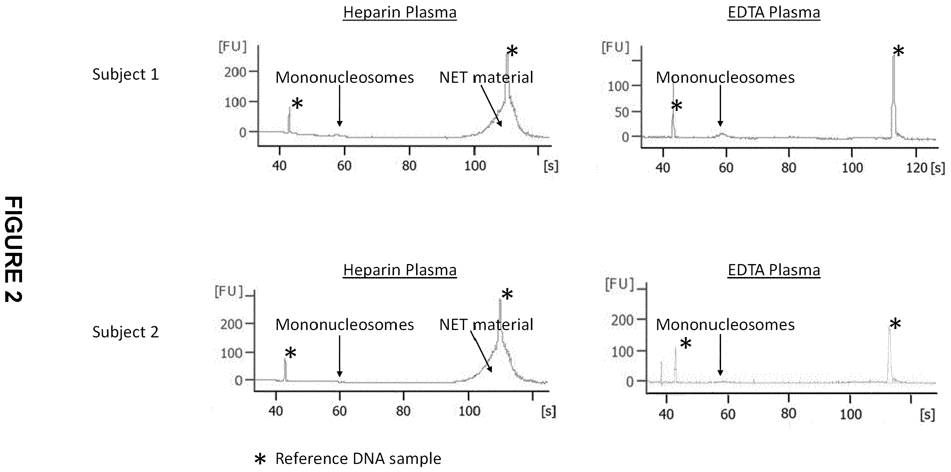

**Figure 2****.** Bioanalyzer electrophoresis results for NET derived nucleosomes in EDTA plasma and heparin plasma samples taken from 2 healthy volunteers. The EDTA samples contain low levels of both mononucleosomes and NET derived nucleosome material. In contrast, heparin induces NET formation and the heparin plasma samples contain low levels of mononucleosomes (peak at approximately 60 seconds) but high levels of induced NET derived nucleosomes (wide peak at approximately 110 seconds). The narrow peaks at approximately 43 seconds and approximately 110 seconds represent DNA samples added for reference purposes.

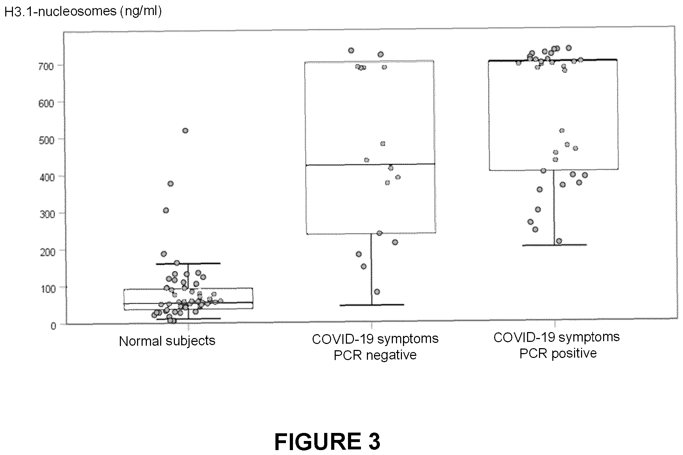

**Figure 3****.** Levels of nucleosomes containing histone isoform H3.1 measured in 50 patients admitted to hospital for symptoms of COVID-19 infection, including 34 symptomatic patients who tested positive for COVID-19 infection by PCR and 16 symptomatic patients who tested negative by PCR, as well as 50 normal subjects displaying no symptoms of disease.

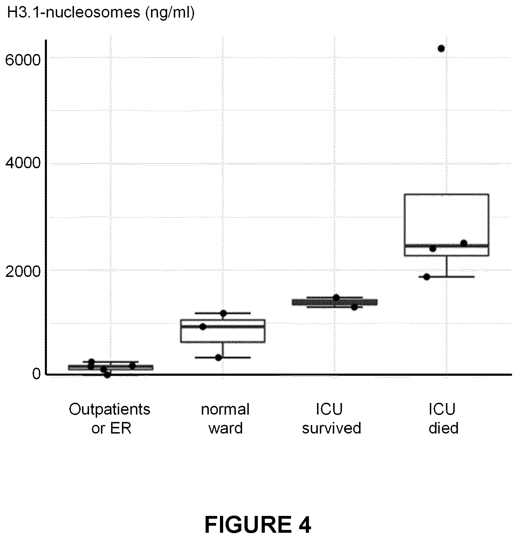

**Figure 4****.** Levels of nucleosomes containing histone isoform H3.1 measured in 15 patients with PCR confirmed COVID-19 infection, including: 5 samples collected from patients attending an outpatient hospital appointment or at presentation at the hospital Emergency Room (ER); 3 patients hospitalized in normal wards; 2 patients hospitalized in an intensive care unit (ICU) who required respiratory support and survived; and 4 patients hospitalized in an ICU who required respiratory support and died.

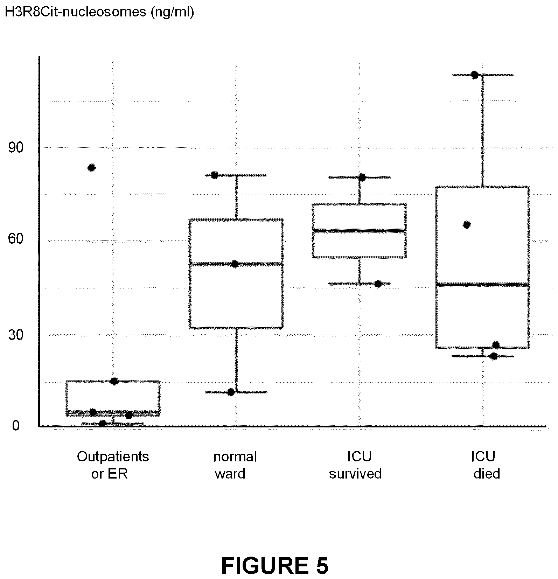

**Figure 5****.** Levels of nucleosomes containing histone modification H3R8Cit measured in 15 patients with PCR confirmed COVID-19 infection, including: 5 samples collected from patients attending an outpatient hospital appointment or at presentation at the hospital ER; 3 patients hospitalized in normal wards; 2 patients hospitalized in an ICU who required respiratory support and survived; and 4 patients hospitalized in an ICU who required respiratory support and died.

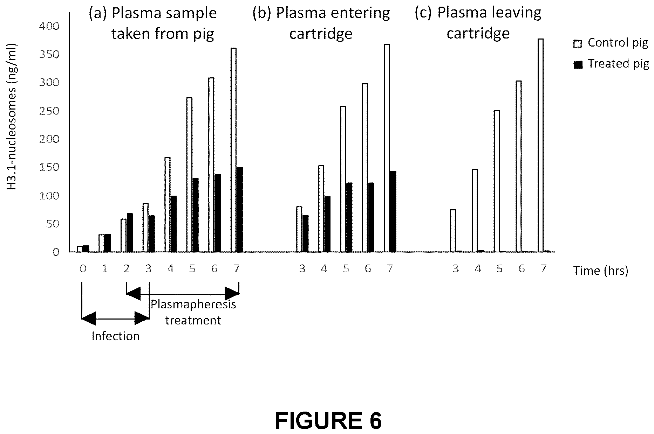

**Figure 6****.** Results from the experiment described in Example 12 showing the mean levels of nucleosomes containing histone isoform H3.1 measured in 16 pigs induced with sepsis placed on plasmapheresis. In 9 pigs, plasma was passed through a cartridge containing NET binders (treated, closed bars) and in 7 pigs, plasma was passed through a control cartridge which did not contain a NETs binder (control, open bars).

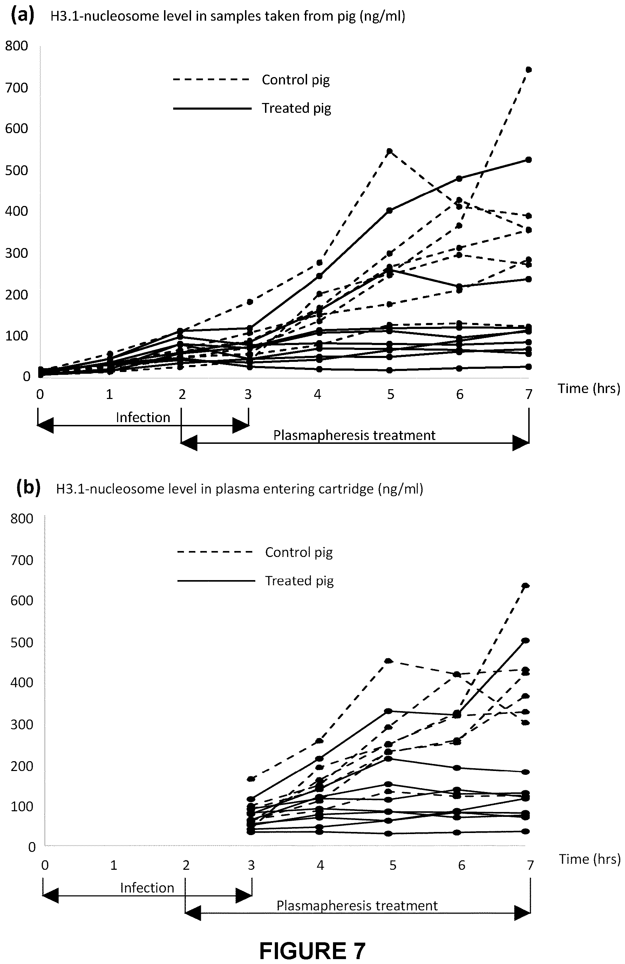

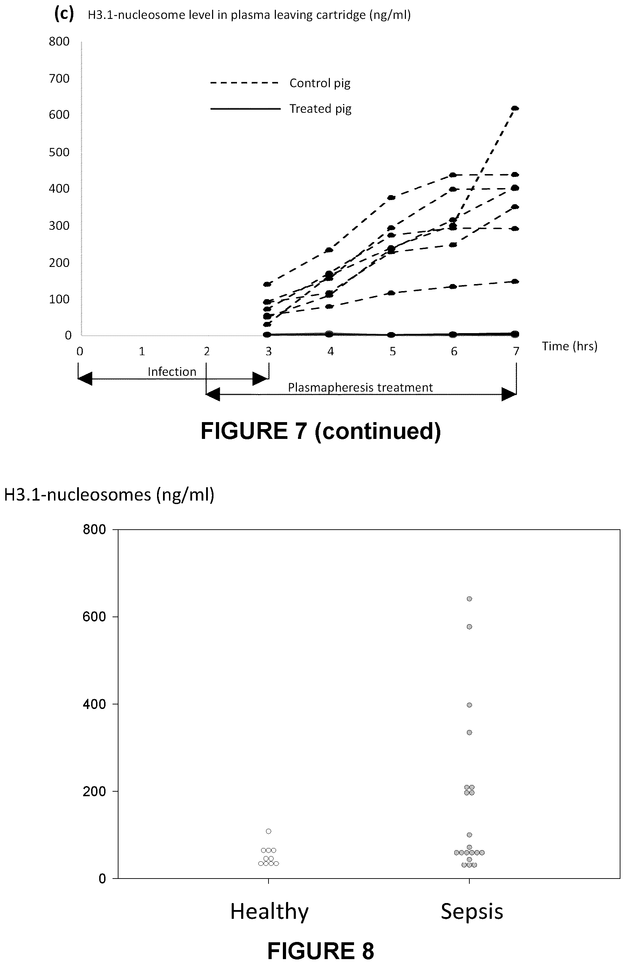

**Figure 7****.** Results from the experiment described in Example 12 and shown in Figure 6, but for levels in individual test subjects.
**Figure 8****.** H3.1-nucleosome levels measured in human subjects diagnosed with sepsis and healthy human subjects.

### SUMMARY OF THE INVENTION

According to a first aspect, there is provided a method of monitoring the progress of a disease in a subject suffering from an infection, comprising:
(i) contacting a body fluid sample obtained from the subject with a binding agent to detect or measure the level of cell free nucleosomes or a component thereof;
(ii) repeating step (i) on one or more occasions; and
(iii) using any changes in the level of cell free nucleosomes or component thereof to monitor the progression of the infection in the subject.

According to a further aspect, there is provided a method of assigning a risk of the development or progression of a medical complication in a subject suffering from an infection, comprising:
(i) contacting a body fluid sample obtained from the subject with a binding agent to detect or measure the level of cell free nucleosomes or a component thereof; and
(ii) using the level of cell free nucleosomes detected to assign the likelihood that a medical complication will develop or progress in said subject.

According to a further aspect, there is provided a method of assigning a risk of an adverse outcome to a subject suffering from an infection, comprising:
(i) contacting a body fluid sample obtained from the subject with a binding agent to detect or measure the level of cell free nucleosomes or a component thereof; and
(ii) using the level of cell free nucleosomes detected to assign the likelihood of an adverse outcome to said subject,
wherein a subject identified with a high likelihood of an adverse outcome is assigned for medical intervention.

According to a further aspect, there is provided a method of selecting a subject suffering from an infection, who is in need of medical treatment for a medical complication of the infection, comprising:
(i) contacting a body fluid sample obtained from the subject with a binding agent to detect or measure the level of cell free nucleosomes or a component thereof; and
(ii) using the level of cell free nucleosomes detected to indicate the presence, progression or development of a medical complication in need of treatment in said subject.

In preferred embodiments the infection is a respiratory influenza or coronavirus infection and the medical complication is ARS, ARDS or SARS or pneumonia. Therefore in one embodiment there is provided a method of detecting a subject in need of medical treatment for pneumonia, ARS, ARDS or SARS, comprising:
(i) contacting a body fluid sample obtained from the subject with a binding agent to detect or measure the level of cell free nucleosomes or a component thereof; and
(ii) using the level of cell free nucleosomes as an indicator that the subject is in need of medical treatment for pneumonia, ARS, ARDS or SARS.

In preferred embodiments the infection is a respiratory influenza or coronavirus infection and the medical complication is ARS, ARDS, SARS or pneumonia.

In other preferred embodiments the infection is sepsis. Therefore in one embodiment there is provided a method of detecting a subject in need of medical treatment for sepsis or septic shock, comprising:
(i) contacting a body fluid sample obtained from the subject with a binding agent to detect or measure the level of cell free nucleosomes or a component thereof; and
(ii) using the level of cell free nucleosomes as an indicator that the subject is in need of medical treatment for sepsis or septic shock.

According to a further aspect of the invention, there is provided a method of monitoring an infection in a subject, comprising:
(i) contacting a body fluid sample obtained from the subject with a binding agent to detect or measure the level of cell free nucleosomes or a component thereof;
(ii) repeating the detection or measurement of the level of cell free nucleosomes or a component thereof in a body fluid obtained from the subject on one or more occasions;
(iii) using any changes in the level of cell free nucleosomes or component thereof to monitor the progression of the infection in the subject.

### DETAILED DESCRIPTION

Nucleosomes are released into the circulation on fragmentation of chromatin on cell death. Many infections, such as viral infections, initiate cell death through a variety of mechanisms (cell binding and entry, endosomal TLR3 activation and gene expression) thereby increasing the number of circulating nucleosomes in the blood (Danthi et al., Annu. Rev. Virol. (2016) 3: 533-53). In addition, infections can induce NETosis whereby post-translational histone modifications, such as acetylation or hypercitrullination of histones H3 and H4 (Wang Y et al., J. Cell Biol. (2009) 184(2): 205-213), promote decondensation of chromatin which is released into circulation together as a first line response to infection. However, extracellular nucleosomes and neutrophil extracellular traps (NETs) can cause severe complications if not cleared rapidly. For example, nucleosome binding to the glomerular membrane is associated with kidney damage in lupus (Kalaaji et al., Kidney Int. (2007) 71(7): 665-672), whilst NETs have been shown to intensify pulmonary injury during viral pneumonia (Ashar et al., Am. J. Pathol. (2018) 188(1): 135-148). Indeed, host directed NET toxicity is associated with respiratory distress, occlusion of narrow airways, endothelial and epithelial cell damage, inflammatory response and thrombus formation and other pathologies (Marcos et al., Nat. Med. (2010) 16: 1018-23; Hoeksema et al., Future Microbiol. (2016) 11: 441-53).

Most subjects infected with influenza or coronavirus experience mild illness. However, some population subgroups, including elderly persons aged over 60 years and persons with an underlying medical condition such as diabetes, chronic lung conditions and particularly chronic cardiac conditions, are at risk of severe effects including ARS, SARS, pneumonia and death. The exact mechanism by which influenza or coronavirus infection leads to complications including pneumonia is not clear, but it is thought to be caused by a hyperimmune reaction to the viral infection in which excessive NETs contribute to acute injury of the lung leading to pneumonia and, in the worst cases, death.

The present invention utilises elevated levels of cell free nucleosomes, including NETs, to predict severity of disease and outcome in infectious disease.

Therefore, according to one aspect, there is provided a method of assigning a risk of an adverse outcome to a subject suffering from an infection, comprising:
(i) contacting a body fluid sample obtained from the subject with a binding agent to detect or measure the level of cell free nucleosomes or a component thereof; and
(ii) using the level of cell free nucleosomes detected to assign the likelihood of an adverse outcome to said subject. The method may be used so that a subject identified with a high likelihood of an adverse outcome is assigned for medical intervention.

In one embodiment, there is provided a method of assigning a risk of an adverse outcome to a subject suffering from an infection, comprising:
(i) contacting a body fluid sample obtained from the subject with a binding agent to detect or measure the level of neutrophil extracellular trap material or a component thereof; and
(ii) using the level of neutrophil extracellular trap material detected to assign the likelihood of an adverse outcome to said subject.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller, Methods Mol. Biol. (2007) 361: 25-62).

References to "nucleosome" may refer to "cell free nucleosome" when detected in body fluid samples. It will be appreciated that the term cell free nucleosome throughout this document is intended to include any cell free chromatin fragment that includes one or more nucleosomes.

It will be understood that the cell free nucleosome may be detected by binding to a component thereof. The term "component thereof' as used herein refers to a part of the nucleosome, *i.e.* the whole nucleosome does not need to be detected. The component of the cell free nucleosomes may be selected from the group consisting of: a histone protein (*i.e.* histone H1, H2A, H2B, H3 or H4), a histone post-translational modification, a histone variant or isoform, a protein bound to the nucleosome (*i.e.* a nucleosome-protein adduct), a DNA fragment associated with the nucleosome and/or a modified nucleotide associated with the nucleosome. For example, the component thereof may be histone (isoform) H3.1 or histone H1 or DNA.

Methods and uses of the invention may measure the level of (cell free) nucleosomes *per se.* References to "nucleosomes *per se*" refers to the total nucleosome level or concentration present in the sample, regardless of any epigenetic features the nucleosomes may or may not include. Detection of the total nucleosome level typically involves detecting a histone protein common to all nucleosomes, such as histone H4. Therefore, nucleosomes *per se* may be measured by detecting a core histone protein, such as histone H4. As described herein, histone proteins form structural units known as nucleosomes which are used to package DNA in eukaryotic cells.

Normal cell turnover in adult humans involves the creation by cell division of a huge number of cells daily and the death of a similar number, mainly by apoptosis. During the process of apoptosis chromatin is broken down into mononucleosomes and oligonucleosomes which are released from the cells. Under normal conditions the levels of circulating nucleosomes found in healthy subjects is reported to be low. Elevated levels are found in subjects with a variety of conditions including many cancers, auto-immune diseases, inflammatory conditions, stroke and myocardial infarction (Holdenreider & Stieber, Crit. Rev. Clin. Lab. Sci. (2009) 46(1): 1-24).

Previous nucleosome ELISA methods were used primarily in cell culture, usually as a method to detect apoptosis (Salgame et al., Nucleic Acids Res. (1997) 25(3): 680-681; Holdenrieder *et al.* (2001) *supra*; van Nieuwenhuijze et al., Ann. Rheum. Dis. (2003) 62: 10-14), but are also used for the measurement of circulating cell free nucleosomes in serum and plasma (Holdenrieder *et al.* (2001)). Cell free serum and plasma nucleosome levels released into the circulation by dying cells have been measured by ELISA methods in studies of a number of different cancers to evaluate their use as a potential biomarker.

The cell free nucleosome may be mononucleosomes, oligonucleosomes, a constituent part of a larger chromatin fragment or a constituent part of a NET or a mixture thereof.

Mononucleosomes and oligonucleosomes can be detected by Enzyme-Linked ImmunoSorbant Assay (ELISA) and several methods have been reported (*e.g*. Salgame *et al.* (1997); Holdenrieder *et al.* (2001); van Nieuwenhuijze *et al.* (2003)). These assays typically employ an anti-histone antibody (for example anti-H2B, anti-H3 or anti-H1, H2A, H2B, H3 and H4) as capture antibody and an anti-DNA or anti-H2A-H2B-DNA complex antibody as detection antibody.

Circulating nucleosomes are not a homogeneous group of protein-nucleic acid complexes. Rather, they are a heterogeneous group of chromatin fragments originating from the digestion of chromatin on cell death and include an immense variety of epigenetic structures including particular histone isoforms (or variants), post-translational histone modifications, nucleotides or modified nucleotides, and protein adducts. It will be clear to those skilled in the art that an elevation in nucleosome levels will be associated with elevations in some circulating nucleosome subsets containing particular epigenetic signals including nucleosomes comprising particular histone isoforms (or variants), comprising particular post-translational histone modifications, comprising particular nucleotides or modified nucleotides and comprising particular protein adducts. Assays for these types of chromatin fragments are known in the art (for example, see WO 2005/019826, WO 2013/030579, WO 2013/030578, WO 2013/084002 which are herein incorporated by reference).

A number of proteins occur in NETs that are adducted directly or indirectly to nucleosomes. These proteins include, without limitation, myeloperoxidase (MPO), neutrophil elastase (NE), lactotransferrin, azurocidin, cathepsin G, leukocyte proteinase 3, lysozyme C, neutrophil defensin 1, neutrophil defensin 3, myeloid cell nuclear differentiation antigen, S100 calcium-binding protein A8, S100 calcium-binding protein A9, S100 calcium-binding protein A12, actin β, actin γ, alpha-actin, plastin-2, cytokeratin-10, catalase, alpha-enolase and transketolase (Urban et al., PLOS Pathogens. (2009) 10: e1000639). Any nucleosome-protein adduct that occurs in NETs is a useful adduct for the detection of elevated levels of NETs in methods of the invention. C-reactive protein (CRP) may also be adducted to nucleosomes in NETs and nucleosome-CRP adduct is therefore a useful adduct for the detection of elevated levels of NETs in methods of the invention.

In preferred embodiments of the invention the adduct used is a MPO-nucleosome adduct or a NE-nucleosome adduct.

In one embodiment, the component of the cell free nucleosome comprises an epigenetic feature of the cell free nucleosome.

The biomarker used in the methods of the invention may be the level of cell free nucleosomes *per se* and/or an epigenetic feature of a cell free nucleosome. It will be understood that the terms "epigenetic signal structure" and "epigenetic feature" are used interchangeably herein. They refer to particular features of the nucleosome that may be detected. In one embodiment, the epigenetic feature of the nucleosome is selected from the group consisting of: a post-translational histone modification, a histone isoform, a modified nucleotide and/or proteins bound to a nucleosome in a nucleosome-protein adduct.

In one embodiment, the epigenetic feature of the nucleosome comprises one or more histone variants or isoforms. The epigenetic feature of the cell free nucleosome may be a histone isoform, such as a histone isoform of a core nucleosome, in particular a histone H3 isoform. The term "histone variant" and "histone isoform" may be used interchangeably herein. The structure of the nucleosome can also vary by the inclusion of alternative histone isoforms or variants which are different gene or splice products and have different amino acid sequences. Many histone isoforms are known in the art. Histone variants can be classed into a number of families which are subdivided into individual types. The nucleotide sequences of a large number of histone variants are known and publicly available for example in the National Human Genome Research Institute NHGRI Histone Database (Mariño-Ramírez et al. The Histone Database: an integrated resource for histones and histone fold-containing proteins. Database Vol.2011. and http://genome.nhgri.nih.gov/histones/complete.shtml), the GenBank (NIH genetic sequence) Database, the EMBL Nucleotide Sequence Database and the DNA Data Bank of Japan (DDBJ). For example, variants of histone H2 include H2A1, H2A2, mH2A1, mH2A2, H2AX and H2AZ. In another example, histone isoforms of H3 include H3.1, H3.2, H3.3 and H3t.

In one embodiment, the histone isoform is H3.1.

The structure of nucleosomes can vary by post translational modification (PTM) of histone proteins. PTM of histone proteins typically occurs on the tails of the core histones and common modifications include acetylation, methylation or ubiquitination of lysine residues as well as methylation or citrullination of arginine residues and phosphorylation of serine residues and many others. Many histone modifications are known in the art and the number is increasing as new modifications are identified (Zhao and Garcia (2015) Cold Spring Harb Perspect Biol, 7: a025064). Therefore, in one embodiment, the epigenetic feature of the cell free nucleosome may be a histone post translational modification (PTM). The histone PTM may be a histone PTM of a core nucleosome, *e.g*. H3, H2A, H2B or H4, in particular H3, H2A or H2B. In particular, the histone PTM is a histone H3 PTM. Examples of such PTMs are described in WO 2005/019826.

For example, the post translational modification may include acetylation, methylation, which may be mono-, di-or tri-methylation, phosphorylation, ribosylation, citrullination, ubiquitination, hydroxylation, glycosylation, nitrosylation, glutamination and/or isomerisation (see Ausio (2001) Biochem Cell Bio 79: 693). In one embodiment, the histone PTM is selected from citrullination or ribosylation. In a further embodiment, the histone PTM is H3 citrulline (H3cit) or H4 citrulline (H4cit). In a yet further embodiment, the histone PTM is H3cit.

In one embodiment, the histone PTM is ribosylation, also referred to as ADP-ribosylation. Post-translational histone ADP-ribosylation of nucleosomes occupying promoters of inflammatory response markers in macrophages is stimulated by exposure to lipopolysaccharides leading to elevated transcription and may have antiviral properties. Moreover, all members of the Coronavirus family contain a highly conserved macrodomain within non-structural protein 3 (nsp3) that regulates post-translational ADP-ribosylation by enzymatic removal of covalently attached ADP-ribose from protein targets. Recombinant severe acute respiratory syndrome coronavirus (SARS-CoV) strains containing mutated macrodomains with reduced nsp3 de-ADP-ribosylation activity, are less infective and elicit an early, enhanced interferon (IFN), interferon-stimulated gene (ISG), and proinflammatory cytokine response. Therefore, altered levels of circulating ADP-ribosylated nucleosomes released from macrophages are expected to be useful in methods of the invention.

A group or class of related histone post translational modifications (rather than a single modification) may also be detected. A typical example, without limitation, would involve a 2-site immunoassay employing one antibody or other selective binder directed to bind to nucleosomes and one antibody or other selective binder directed to bind the group of histone modifications in question. Examples of such antibodies directed to bind to a group of histone modifications would include, for illustrative purposes without limitation, anti-pan-acetylation antibodies (*e.g*. a Pan-acetyl H4 antibody [H4panAc]), anti-citrullination antibodies or anti-ubiquitin antibodies.

In one embodiment, the epigenetic feature of the nucleosome comprises one or more DNA modifications. In addition to the epigenetic signalling mediated by nucleosome histone isoform and PTM composition, nucleosomes also differ in their nucleotide and modified nucleotide composition. Some nucleosomes may comprise more 5-methylcytosine residues (or 5-hydroxymethylcytosine residues or other nucleotides or modified nucleotides) than other nucleosomes. In one embodiment, the DNA modification is selected from 5-methylcytosine or 5-hydroxymethylcytosine.

In one embodiment, the epigenetic feature of the nucleosome comprises one or more protein-nucleosome adducts or complexes. A further type of circulating nucleosome subset is nucleosome protein adducts. It has been known for many years that chromatin comprises a large number of non-histone proteins bound to its constituent DNA and/or histones. These chromatin associated proteins are of a wide variety of types and have a variety of functions including transcription factors, transcription enhancement factors, transcription repression factors, histone modifying enzymes, DNA damage repair proteins and many more. These chromatin fragments including nucleosomes and other non-histone chromatin proteins or DNA and other non-histone chromatin proteins are described in the art.

In one embodiment, the protein adducted to the nucleosome (and which therefore may be used as a biomarker) is selected from: a transcription factor, a High Mobility Group Protein or chromatin modifying enzyme. References to "transcription factor" refer to proteins that bind to DNA and regulate gene expression by promoting (*i.e*. activators) or suppressing (*i.e*. repressors) transcription. Transcription factors contain one or more DNA-binding domains (DBDs), which attach to specific sequences of DNA adjacent to the genes that they regulate. All of the circulating nucleosomes and nucleosome moieties, types or subgroups described herein may be useful in the present invention.

It will be understood that more than one epigenetic feature of cell free nucleosomes may be detected in methods and uses of the invention. Multiple biomarkers may be used as a combined biomarker. Therefore, in one embodiment, the use comprises more than one epigenetic feature of cell free nucleosomes as a combined biomarker. The epigenetic features may be the same type (*e.g*. PTMs, histone isoforms, nucleotides or protein adducts) or different types (*e.g*. a PTM in combination with a histone isoform). For example, a post-translational histone modification and a histone variant may be detected (*i.e*. more than one type of epigenetic feature is detected). Alternatively, or additionally, more than one type of post-translational histone modification is detected, or more than one type of histone isoform is detected. In one aspect, the use comprises a post-translational histone modification and a histone isoform as a combined biomarker in a sample, for the diagnosis, detection, treatment selection, prognostication or monitoring of an infection. In one embodiment, the combined biomarker is H3.1 and H3cit. In an alternative embodiment, the combined biomarker is H3.1 and H4cit.

The term "biomarker" means a distinctive biological or biologically derived indicator of a process, event, or condition. Biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

Biomarkers are also useful as companion diagnostic products for the selection of patients suitable for treatment by a particular therapy. We have demonstrated herein that tests for circulating nucleosome levels, or levels of nucleosomes containing particular epigenetic signals or structures, are useful companion products to therapies for NETs or NETosis related diseases.

The methods of the invention are directed to assigning the patient with a risk of an adverse outcome. Adverse outcomes include mortality and/or an acute event requiring immediate medical care, for example, hospitalisation (*i.e*. hospital treatment) and/or surgery. For many patients, infections are overcome by their own immune system without requiring medical intervention. However, in a number of patients, infections can proceed or increase in severity without being overcome by the immune system or the patient's own immune response to an infection may lead to an adverse outcome. For example, an adverse outcome may include an acute coronary or cardiac event (such as a myocardial infarction and/or stroke), acute multi- or single-organ failure (such as renal failure, liver failure and/or heart failure), onset of a debilitating acute condition and/or an acute respiratory condition (such as pneumonia, hypoventilation/bradypnea, acute respiratory distress syndrome (ARDS) severe acute respiratory syndrome (SARS), bronchiolitis and/or bronchitis). Thus, in one embodiment, the methods described herein assign a patient or subject with a risk of developing an acute respiratory condition. In a further embodiment, the acute respiratory condition is pneumonia. In a further embodiment, the acute respiratory condition is hypoventilation/bradypnea. In a yet further embodiment, the acute respiratory condition is acute respiratory distress syndrome (ARDS) and/or severe acute respiratory syndrome (SARS).

Assigning the patient with a risk of an adverse outcome may assign a near- or short-term risk or may assign a medium-term risk. A near- or short-term risk includes wherein the patient may develop an adverse outcome within 30 days, such as within 2 weeks or 14 days, within 1 week or 7 days or within 5 days or less of presentation of symptoms or of a positive diagnosis. Such near- or short-term risk may also include wherein the patient may develop an adverse outcome within 30 days, such as within 2 weeks or 14 days, within 1 week or 7 days or within 5 days or less of performing the methods described herein. An example of a short term risk includes the development of NETs related complications to a COVID infection requiring hospital treatment. A medium-term risk includes wherein the patient may develop an adverse outcome more than 30 days after presentation of symptoms, a positive diagnosis and/or the performing of methods as described herein. An example of a medium term risk includes the development of so called long-COVID wherein the effects of a COVID infection may continue for many months. Thus, in one embodiment, the methods described herein assign a patient or subject with a risk of developing an adverse outcome within 2 weeks, or 14 days, of presentation of symptoms or a positive diagnosis. In a further embodiment, the methods described herein assign a risk of developing an adverse outcome within 1 week, or 7 days, of presentation of symptoms or a positive diagnosis. In a yet further embodiment, the methods described herein assign a risk of developing an adverse outcome within 5 days of presentation of symptoms or a positive diagnosis.

Therefore, in another aspect of the invention, there is provided a method of identifying a subject with an infection requiring hospital treatment, comprising:
(i) contacting a body fluid sample obtained from the subject with a binding agent to detect or measure the level of cell free nucleosomes or a component thereof; and
(ii) using the level of cell free nucleosomes detected to determine if the subject should be admitted to hospital for treatment.

It will be understood that methods of the invention may also be used to identify patients who do not require hospital treatment, i.e. using the level of cell free nucleosomes detected to determine if the subject should not be admitted to hospital for treatment. This mode of the invention would help to identify patients who can be discharged early if they have already been admitted to hospital.

Methods and uses described herein may be tested in body fluid samples, in particular blood, serum or plasma samples. Preferably, plasma samples are used. Plasma samples may be collected in collection tubes containing one or more anticoagulants such as ethylenediamine tetraacetic acid (EDTA), heparin, or sodium citrate, in particular EDTA.

### Infections

The methods of the current invention find particular use in managing infectious outbreaks. Infections can be caused by different pathogens and environmental factors. In one embodiment, the infection is a viral, bacterial, fungal or microbial infection. Bacterial infections may include mycobacterial, pneumococcal and influenzae infections, such as infections (*e.g*. pneumonia) caused by Streptococcus pneumoniae, Escherichia coli, Mycobacterium tuberculosis, Haemophilus influenzae and Staphylococcus aureus. In a further embodiment, the infection is a viral infection. Viral infections may include infections caused by respiratory syncytial virus (RSV), influenza type A, influenza type B and coronaviruses (*e.g*. COVID-19).

The infection can be defined by the tissue affected by the disease. For example, the disease may affect the heart, brain, kidneys, liver, pancreas, lungs and/or blood and the infection may be a bacterial, viral, fungal or microbial infection known to commonly affect such tissues or organs. In one embodiment, the infection is a respiratory tract infection. According to this embodiment, the infection affects the lungs, upper and/or lower respiratory tract.

Other tissues which may be affected by the disease include peripheral tissues such as limbs, hands and feet and the infection may be a bacterial infection (*e.g*. gangrene). In one embodiment, the infection and/or disease may affect multiple tissues or organs simultaneously. For example, the infection may be a bacterial infection of a limb, hand or foot and the disease may also affect the blood (*e.g*. sepsis). In one embodiment, the infection is sepsis. In another example, the disease may be cardiac or coronary failure and other tissues or organs affected by the disease may include the kidneys and renal system and/or the brain (*e.g*. stroke). In a yet further example, the disease may affect the lungs or the infection may be a respiratory tract infection and other tissues or organs affected may include the heart, coronary system and/or brain (*e.g*. heart failure, myocardial infarction and/or stroke).

In one embodiment, circulating nucleosome levels are measured in a sample taken from a subject suffering from an infection to determine the prognosis of the disease. In another embodiment, circulating nucleosome levels are measured in multiple samples taken at intervals from a subject suffering from an infection to monitor the progress of the disease and/or to assess the efficacy of treatment.

In a further embodiment, circulating nucleosome levels are measured in a sample taken from a subject suffering from sepsis or septic shock, in particular to assess the prognosis of the disease. Further measurements on multiple samples taken at intervals from a subject suffering from sepsis or septic shock may be made to monitor the progress of the disease and/or to assess the efficacy of treatment.

In one embodiment, the respiratory tract infection is selected from: influenza, pneumonia and severe acute respiratory syndrome (SARS). SARS is a respiratory infection caused by the SARS coronavirus (SARS-CoV) and other, related coronaviruses are known (*e.g*. COVID-19 (also known as SARS-CoV-2 and previously as 2019-nCoV)). It is known to cause fever flu-like symptoms, cough and lethargy and can lead to pneumonia (*e.g*. direct viral pneumonia or secondary bacterial pneumonia).

The emergence and rapid progression of COVID-19 to pandemic status places severe strain on international health care services. Predictions of infectivity range as high as 70-80% of national populations. It appears that whilst most people will experience mild symptoms, double digit percentages of those infected may be severely affected.

Identifying COVID-19 positive individuals at high risk of severe reaction or a complication, including pneumonia, would allow triaging and facilitate allocation of strained medical resources including critical care beds and ventilators, until herd immunity is established, protecting communities from future widespread outbreaks. Therefore, in a preferred embodiment there is provided a method of identifying a subject infected with an influenza or coronavirus infection requiring medical treatment, comprising:
(i) contacting a body fluid sample obtained from the subject with a binding agent to detect or measure the level of cell free nucleosomes or a component thereof; and
(ii) using the level of cell free nucleosomes detected to determine if the subject requires medical treatment.

### Diagnosis and monitoring methods

According to a further aspect, there is provided a method of monitoring the severity of an infection in a subject, comprising:
(i) contacting a body fluid sample obtained from the subject with a binding agent to detect or measure the level of cell free nucleosomes or a component thereof;
(ii) repeating the detection or measurement of the level of cell free nucleosomes or a component thereof in a body fluid obtained from the subject on one or more occasions;
(iii) using any changes in the level of cell free nucleosomes or component thereof to monitor the progression of the infection in the subject.

According to a further aspect, there is provided a method for monitoring the progression of an infection in a subject having or suspected of having an infection, or being predisposed to a poor prognosis on infection, which comprises the steps of:
(i) contacting a sample obtained from the subject with a binding agent to detect or measure the level of cell free nucleosomes; and
(ii) comparing the level of cell free nucleosomes detected with an earlier sample taken from said subject to monitor the progression of the infection.

According to a further aspect, there is provided a method of monitoring the progress of a disease in a subject suffering from an infection, comprising:
(i) contacting a body fluid sample obtained from the subject with a binding agent to detect or measure the level of cell free nucleosomes or a component thereof;
(ii) repeating step (i) on one or more occasions; and
(iii) using any changes in the level of cell free nucleosomes or component thereof to monitor the progression of the infection in the subject.

If a subject is determined to not have an infection or to have a mild infection, then the invention may still be used for the purposes of monitoring disease progression for future development of a medical complication. For example, if the method comprises a sample from a subject determined to have a mild infection, then the biomarker level measurements can be repeated at another time point to establish if the biomarker level has changed.

Detecting and/or quantifying may be performed directly on the purified or enriched nucleosome sample, or indirectly on an extract therefrom, or on a dilution thereof. Quantifying the amount of the biomarker present in a sample may include determining the concentration of the biomarker present in the sample. Uses and methods of detecting, monitoring and of diagnosis according to the invention described herein are useful to confirm the existence of a disease, to monitor development of the disease by assessing onset and progression, or to assess amelioration or regression of the disease. Uses and methods of detecting, monitoring and of diagnosis are also useful in methods for assessment of clinical screening, prognosis, choice of therapy, evaluation of therapeutic benefit, *i.e.* for drug screening and drug development.

In one embodiment the disease is a condition involving pathological clinical complications of high levels of NETs or NETosis.

The detection or measurement may comprise an immunoassay, immunochemical, mass spectroscopy, chromatographic, chromatin immunoprecipitation or biosensor method. In particular, detection and/or measurement may comprise a 2-site immunoassay method for nucleosome moieties. Such a method is preferred for the measurement of nucleosomes or nucleosome incorporated epigenetic features *in situ* employing two anti-nucleosome binding agents or an anti-nucleosome binding agent in combination with an anti-histone modification or anti-histone variant or anti-DNA modification or anti-adducted protein detection binding agent. Also, detection and/or measurement may comprise a 2-site immunoassay, for example employing combinations of a labelled or immobilized: anti-nucleosome, anti-histone modification, anti-histone variant/isoform, anti-DNA modification or anti-adducted protein binding agent.

The inventors herein used a 2-site immunoassay for H3.1-nucleosomes employing an immobilized anti-histone H3.1 antibody directed to bind to an epitope around amino acids 30-33 of the histone H3.1 protein to capture clipped and non-clipped nucleosomes, together with a labelled anti-nucleosome antibody directed to bind to a epitope present in intact nucleosomes but not present on isolated (free) histone or DNA nucleosome components. This type of epitope may be referred to as a "conformational nucleosome epitope" herein because it requires the native three-dimensional configuration of the target nucleosome to be intact.

H3R8Cit nucleosome measurements described herein were performed using a 2-site immunoassay employing an immobilized antibody directed to bind to nucleosomes citrullinated at arginine 8 of histone H3, together with the same labelled anti-nucleosome antibody directed to bind to a conformational nucleosome epitope.

In one embodiment, the method of detection or measurement comprises contacting the body fluid sample with a solid phase comprising a binding agent that detects cell free nucleosomes or a component thereof, and detecting binding to said binding agent.

In one embodiment, the method of detection or measurement comprises: (i) contacting the sample with a first binding agent which binds to an epigenetic feature of a cell free nucleosome; (ii) contacting the sample bound by the first binding agent in step (i) with a second binding agent which binds to cell free nucleosomes; and (iii) detecting or quantifying the binding of the second binding agent in the sample.

In another embodiment, the method of detection or measurement comprises: (i) contacting the sample with a first binding agent which binds to cell free nucleosomes; (ii) contacting the sample bound by the first binding agent in step (i) with a second binding agent which binds to an epigenetic feature of the cell free nucleosome; and (iii) detecting or quantifying the binding of the second binding agent in the sample.

Detecting or measuring the level of the biomarker(s) may be performed using one or more reagents, such as a suitable binding agent. For example, the one or more binding agents may comprise a ligand or binder specific for the desired biomarker, *e.g*. nucleosomes or component part thereof, an epigenetic feature of a nucleosome, a structural/shape mimic of the nucleosome or component part thereof, optionally in combination with one or more interleukins.

It will be clear to those skilled in the art that the terms "antibody", "binder" or "ligand" as used herein are not limiting but are intended to include any binder capable of binding to particular molecules or entities and that any suitable binder can be used in the method of the invention. It will also be clear that the term "nucleosomes" is intended to include mononucleosomes, oligonucleosomes, NETs and any protein-DNA chromatin fragments that can be analysed in fluid media.

Methods of detecting biomarkers are known in the art. The reagents may comprise one or more ligands or binders, for example, naturally occurring or chemically synthesised compounds, capable of specific binding to the desired target. A ligand or binder may comprise a peptide, an antibody or a fragment thereof, or a synthetic ligand such as a plastic antibody, or an aptamer or oligonucleotide, capable of specific binding to the desired target. The antibody can be a monoclonal antibody or a fragment thereof. It will be understood that if an antibody fragment is used then it retains the ability to bind the biomarker so that the biomarker may be detected (in accordance with the present invention). A ligand/binder may be labelled with a detectable marker, such as a luminescent, fluorescent, enzyme or radioactive marker; alternatively or additionally a ligand according to the invention may be labelled with an affinity tag, *e.g.* a biotin, avidin, streptavidin or His (*e.g*. hexa-His) tag. Alternatively, ligand binding may be determined using a label-free technology for example that of ForteBio Inc.

The term "detecting" or "diagnosing" as used herein encompasses identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, *i.e.* for drug screening and drug development.

Methods of the invention may involve normalisation of marker levels. For example, the level of cell free nucleosomes containing a particular epigenetic feature may be normalised against the level of nucleosomes per se (or some other type of nucleosomes or parameter) to express the level as a proportion of nucleosomes containing the feature. For example, to express the level of citrullinated nucleosomes as the proportion of nucleosomes that are citrullinated.

In one embodiment, the method described herein is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

In monitoring methods, test samples may be taken on two or more occasions. The method may further comprise comparing the level of the biomarker(s) present in the test sample with one or more control(s) and/or with one or more previous test sample(s) taken earlier from the same test subject, *e.g*. prior to commencement of therapy, and/or from the same test subject at an earlier stage of therapy. The method may comprise detecting a change in the nature or amount of the biomarker(s) in test samples taken on different occasions.

A change in the level of the biomarker in the test sample relative to the level in a previous test sample taken earlier from the same test subject may be indicative of a beneficial effect, *e.g*. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (*e.g*. in animal models). These monitoring methods can be incorporated into screens for new drug substances and combinations of substances.

In a further embodiment the monitoring of more rapid changes due to fast acting therapies may be conducted at shorter intervals of hours or days.

Diagnostic or monitoring kits (or panels) are provided for performing methods of the invention. Such kits will suitably comprise one or more ligands for detection and/or quantification of the biomarker according to the invention, and/or a biosensor, and/or an array as described herein, optionally together with instructions for use of the kit.

A further aspect of the invention is a kit for detecting the presence of an infection, comprising a biosensor capable of detecting and/or quantifying one or more of the biomarkers as defined herein. As used herein, the term "biosensor" means anything capable of detecting the presence of the biomarker. Examples of biosensors are described herein. Biosensors may comprise a ligand binder or ligands, as described herein, capable of specific binding to the biomarker. Such biosensors are useful in detecting and/or quantifying a biomarker of the invention.

Suitably, biosensors for detection of one or more biomarkers combine biomolecular recognition with appropriate means to convert detection of the presence, or quantitation, of the biomarker in the sample into a signal. Biosensors can be adapted for "alternate site" diagnostic testing, *e.g*. in the ward, outsubjects' department, surgery, home, field and workplace. Biosensors to detect one or more biomarkers of the invention include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers.

Biomarkers for detecting the presence of a disease are essential targets for discovery of novel targets and drug molecules that retard or halt progression of the disorder. As the level of the biomarker is indicative of disorder and of drug response, the biomarker is useful for identification of novel therapeutic compounds in *in vitro* and/or *in vivo* assays. Biomarkers described herein can be employed in methods for screening for compounds that modulate the activity of the biomarker.

Thus, in a further aspect of the invention, there is provided the use of a binder or ligand, as described, which can be a peptide, antibody or fragment thereof or aptamer or oligonucleotide directed to a biomarker according to the invention; or the use of a biosensor, or an array, or a kit according to the invention, to identify a substance capable of promoting and/or of suppressing the generation of the biomarker.

The immunoassays described herein include any method employing one or more antibodies or other specific binders directed to bind to the biomarkers defined herein. Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, see for example Salgame *et al.* (1997) and van Nieuwenhuijze *et al.* (2003).

Identifying, detecting and/or quantifying can be performed by any method suitable to identify the presence and/or amount of a specific protein in a biological sample from a subject or a purification or extract of a biological sample or a dilution thereof. In particular, quantifying may be performed by measuring the concentration of the target in the sample or samples. Biological samples that may be tested in a method of the invention include those as defined hereinbefore. The samples can be prepared, for example where appropriate diluted or concentrated, and stored in the usual manner. The present invention finds particular use in plasma samples which may be obtained from the subject.

Identification, detection and/or quantification of biomarkers may be performed by detection of the biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

For example, detecting and/or quantifying can be performed by one or more method(s) selected from the group consisting of: immunoassay, immunochromatography, SELDI (-TOF), MALDI (-TOF), a 1-D gel-based analysis, a 2-D gel-based analysis, Mass spectrometry (MS), reverse phase (RP) LC, size permeation (gel filtration), ion exchange, affinity, HPLC, UPLC and other LC or LC MS-based techniques. Appropriate LC MS techniques include ICAT^{®} (Applied Biosystems, CA, USA), or iTRAQ^{®} (Applied Biosystems, CA, USA). Liquid chromatography (*e.g*. high pressure liquid chromatography (HPLC) or low pressure liquid chromatography (LPLC)), thin-layer chromatography, NMR (nuclear magnetic resonance) spectroscopy could also be used.

Methods involving detection and/or quantification of one or more biomarkers of the invention can be performed on bench-top instruments, or can be incorporated onto disposable, diagnostic or monitoring platforms that can be used in a non-laboratory environment, *e.g*. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine. Therefore, in a further aspect of the invention, there is provided the use of a near patient or point-of-care immunoassay method for the measurement of a biomarker according to the invention. In one embodiment the near patient immunoassay method comprises a point-of-care immunoassay instrument (e.g. the Abbott i-STAT or the LightDeck Diagnostics point-of-care immunoassay instrument). In one embodiment the near patient immunoassay method comprises a lateral flow test. In a preferred embodiment the biomarker is a nucleosome or a nucleosome containing an epigenetic feature.

The identification of biomarkers for a disease state permits integration of diagnostic procedures and therapeutic regimes. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus, by monitoring a biomarker of the invention, subject care can be tailored precisely to match the needs determined by the disorder and the pharmacological profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Biomarker monitoring methods, biosensors, point-of-care tests, lateral flow tests and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

References to "subject" or "patient" are used interchangeably herein. The subject may be a human or an animal subject. In one embodiment, the subject is a human. In one embodiment, the subject is a (non-human) animal. The panels and methods described herein may be performed *in vitro,* or *ex vivo.*

Detecting and/or quantifying may be compared to a cut-off level. Cut-off values can be predetermined by analysing results from multiple patients and controls, and determining a suitable value for classifying a subject as with or without the disease. For example, for diseases where the level of biomarker is higher in patients suffering from the disease, then if the level detected is higher than the cut-off, the patient is indicated to suffer from the disease.

Alternatively, for diseases where the level of biomarker is lower in patients suffering from the disease, then if the level detected is lower than the cut-off, the patient is indicated to suffer from the disease. The advantages of using simple cut-off values include the ease with which clinicians are able to understand the test and the elimination of any need for software or other aids in the interpretation of the test results. Cut-off levels can be determined using methods in the art.

Detecting and/or quantifying may also be compared to a control. It will be clear to those skilled in the art that the control subjects may be selected on a variety of basis which may include, for example, subjects known to be free of the disease or may be subjects with a different disease (for example, for the investigation of differential diagnosis). The "control" may comprise a healthy subject, a non-diseased subject and/or a subject without an infection. The control may also be a subject with the infection displaying no, or mild, symptoms, such as a subject infected with a respiratory virus displaying no, or mild, symptoms. Mild symptoms may include manageable symptoms which do not require hospital intervention and/or intensive medical treatment.

In one embodiment, a subject who tests positive by methods of the invention may be infected with a viral disease and additionally suffers, or goes on to suffer, further medical complications. In contrast, a control subject may also be infected with a viral disease but does not suffer, and does not go on to suffer, medical complications. Comparison with a control is well known in the field of diagnostics. The range of values found in the control group may be used as a normal or healthy or reference range against which the values found for test subjects can be compared. For example, if the reference range is <10 units, then a test value of 5 units would be considered normal, or not in need of treatment, but a value of 11 units would be considered abnormal and indicative of a need for treatment.

Therefore, in one embodiment, the method additionally comprises comparing the level of cell free nucleosomes or component thereof in the body fluid sample of the subject with one or more controls. For example, the method may comprise comparing the level of cell free nucleosomes present in a sample obtained from the subject with the level of cell free nucleosomes present in a sample obtained from a normal subject. The control may be a healthy subject.

In one embodiment, the level of cell free nucleosomes or component thereof is elevated compared to the control.

It will be understood that it is not necessary to measure control levels for comparative purposes on every occasion. For example, for healthy/non-diseased controls, once the 'normal range' is established it can be used as a benchmark for all subsequent tests. A normal range can be established by obtaining samples from multiple control subjects without an infection and testing for the level of biomarker. Results (*i.e*. biomarker levels) for subjects suspected to have an infection can then be examined to see if they fall within, or outside of, the respective normal range. Use of a 'normal range' is standard practice for the detection of disease.

In one embodiment, the method additionally comprises determining at least one clinical parameter for the patient. This parameter can be used in the interpretation of results. Clinical parameters may include any relevant clinical information for example, without limitation, body temperature, gender, weight, Body Mass Index (BMI), smoking status and dietary habits. Therefore, in one embodiment, the clinical parameter is selected from the group consisting of: body temperature, age, sex and body mass index (BMI).

In one embodiment, the method of the invention is performed to identify a subject at high risk of developing a severe reaction to an infection and therefore in need of medical intervention. Such medical intervention may include one or more of the therapies as described herein.

According to another aspect of the invention, there is provided the use of a binding agent in the manufacture of a kit for use in a method of assigning a risk of an adverse outcome to a subject suffering from an infection, comprising:
(i) contacting a body fluid sample obtained from the subject with the binding agent to detect or measure the level of cell free nucleosomes or a component thereof; and
(ii) using the level of cell free nucleosomes detected to assign the likelihood of an adverse outcome to said subject.

According to a further aspect of the invention, there is provided the use of a binding agent in the manufacture of a kit for use in a method of detecting a subject in need of medical treatment for pneumonia, acute respiratory syndrome (ARS) or severe acute respiratory syndrome (SARS), comprising:
(i) contacting a body fluid sample obtained from the subject with the binding agent to detect or measure the level of cell free nucleosomes or a component thereof; and
(ii) using the level of cell free nucleosomes as an indicator that the subject is in need of medical treatment for pneumonia, ARS or SARS.

According to a further aspect of the invention, there is provided the use of a binding agent in the manufacture of a kit for use in a method of detecting a subject in need of medical treatment for sepsis or septic shock, comprising:
(i) contacting a body fluid sample obtained from the subject with the binding agent to detect or measure the level of cell free nucleosomes or a component thereof; and
(ii) using the level of cell free nucleosomes as an indicator that the subject is in need of medical treatment for sepsis or septic shock.

According to a further aspect of the invention, there is provided a method of detecting a subject in need of medical treatment for pneumonia, acute respiratory syndrome (ARS) or severe acute respiratory syndrome (SARS), comprising:
(i) contacting a body fluid sample obtained from the subject with a binding agent to detect or measure the level of cell free nucleosomes or a component thereof; and
(ii) using the level of cell free nucleosomes as an indicator that the subject is in need of medical treatment for pneumonia, ARS or SARS.

According to a further aspect of the invention, there is provided a method of detecting a subject in need of medical treatment for sepsis or septic shock, comprising:
(i) contacting a body fluid sample obtained from the subject with a binding agent to detect or measure the level of cell free nucleosomes or a component thereof; and
(ii) using the level of cell free nucleosomes as an indicator that the subject is in need of medical treatment for sepsis or septic shock.

### Additional biomarkers

The level of cell free nucleosomes may be detected or measured as one of a panel of measurements. The panel may comprise different epigenetic features of the nucleosome as described hereinbefore (*e.g*. a histone isoform and a PTM). Biomarkers useful in a panel test for the detection of severe respiratory infections that require medical intervention include, without limitation, cytokine moieties (particularly interleukins), C-reactive protein, myeloperoxidase, D-Dimer, factor VII-activating protease (FSAP), fibrinogen and fibrin/fibrinogen breakdown products. In one embodiment, the panel comprises C-reactive protein. In one embodiment, the panel comprises one or more cytokines, such as one or more interleukins.

Interleukins (ILs) are a group of cytokines, usually secreted by leukocytes, that act as signal molecules. They have key roles in stimulating immune responses and inflammation. They were first identified in the 1970s and have been designated numerically as more interleukin types have been discovered. Examples of interleukins include, but are not limited to: IL-1, IL-2, IL-3, IL-4, IL-5, IL-6, IL-7, IL-8, IL-9, IL-10, IL-11, IL-12, IL-13, IL-14 and IL-15.

In one embodiment, the one or more interleukins is selected from the group consisting of: Interleukin-6 (IL-6) and Interleukin-12 (IL-12).

The interleukin may be IL-6. Interleukin-6 (IL-6) is a cytokine with a wide variety of biological functions. It is a potent inducer of fever and the acute phase response. The sequence of human IL-6 is known in the art and is described at UniProt Accession No. P05231. In one particular embodiment, the interleukin may be IL-6.

Alternatively, or additionally, the interleukin may be IL-12. Interleukin-12 (IL-12) is a T cell stimulating factor because it stimulates the growth and function of T cells. It is a heterodimeric cytokine comprised of IL-12A and IL-12B. The sequence of human IL-12A is known in the art and is described at UniProt Accession No. P29459 and the sequence of human IL-12B is also known and described at UniProt Accession No. P29460. In one particular embodiment, the interleukin may be IL-12.

In one embodiment, the panel comprises a cell free nucleosome or an epigenetic feature thereof and an interleukin. In another embodiment, the panel comprises an epigenetic feature of a cell free nucleosome and two interleukins. For example, the cell free nucleosome measurement can be combined with more than one interleukin measurement, such as IL-6 and IL-12. In a further embodiment, the epigenetic feature of a cell free nucleosome is selected from a histone isoform, such as H3.1, and a post translationally modified histone, such as H3cit. In a yet further embodiment, the panel of measurements is H3.1, H3cit, H4cit and IL-6.

In one embodiment, the panel comprises C-reactive protein (CRP). CRP is a pentameric protein found in plasma and levels of CRP (whether or not adducted to nucleosomes) increase in plasma in response to inflammation, such as in bacterial, viral, fungal and microbial infections. CRP levels increase following IL-6 secretion by macrophages and T cells and its physiological role is to bind lysophosphatidylcholine expressed on the surface of dead or dying cells in order to activate the complement system via C1q. It also binds to phosphocholine on the surface of some bacteria and enhances phagocytosis. The measurement of CRP levels is useful for determining the progression of disease and the effectiveness of treatments and elevated CRP levels have been shown in patients with increased risk of diabetes, hypertension and cardiovascular disease. Increased CRP levels have also been found in patients with kidney failure and inflammatory bowel disease (IBD, including Crohn's disease and ulcerative colitis) and roughly correlate with coronary heart disease, although as elevated CRP is not directly related to heart disease it is not a specific prognostic marker. Since CRP is increased during inflammation, viral infections, such as SARS or coronavirus (*e.g*. COVID-19) may also lead to increased CRP levels in plasma.

In one embodiment, the panel comprises myeloperoxidase (MPO). MPO is expressed in neutrophil granulocytes and produces hypohalous acids to carry out their antimicrobial activity. It is stored in azurophilic granules and released into the extracellular space during degranulation. The levels of MPO have been shown to be a useful predictor for myocardial infarction and have been combined with measurement of CRP for increased accuracy in predicting myocardial infarction risk in patients. In one embodiment, the panel comprises neutrophil elastase (NE).

Models can be derived using the biomarkers of the invention. Methods for deriving models or algorithms are well known in the art and suitable software packages are available. Typical software tools for this purpose include SPSS (Statistical Package for the Social Sciences) and "R". These software packages provide for linear and non-linear data modelling of clinical data.

It will be clear to those skilled in the art, that any combination of the biomarkers disclosed herein may be used in panels and algorithms for the detection or prediction of a complication to an infection, and that further markers may be added to a panel including these markers.

According to an aspect of the invention there is provided the use of a panel test to detect or predict a complication to an infection in a patient, wherein the panel test comprises reagents to detect measurements of nucleosomes or a component thereof and one or more interleukins, in a sample obtained from the patient. In one embodiment the complication is a NETs associated complication. In one embodiment the complication is ARDS, ARS, SARS or an embolism or thrombotic complication.

### Methods of Treatment

According to a further aspect, there is provided a method of treating an infection in a subject, which comprises the following steps:
(i) detecting or measuring the level of cell free nucleosomes in a sample obtained from the subject;
(ii) using the level measured in step (i) as indicative of the presence and/or severity and/or a medical complication of said infection in the subject; and
(iii) administering a therapy if the subject is determined to have a severe infection or a medical complication in step (ii).

According to a further aspect, there is provided a method of treating an infection in a subject in need thereof, which comprises the step of administering a therapy (e.g. a therapeutic agent) to a subject identified as having differing levels of cell free nucleosomes in a sample obtained from said subject, when compared to the level of cell free nucleosomes in a sample obtained from a control subject. The therapy may include one or more suitable treatments for the condition including without limitation, drugs (e.g. anti-inflammatory drugs, blood thinning or clotting inhibitor drugs, therapeutic anti-NETs antibody drugs, DNase drugs, NETosis inhibitor drugs, anti-bacterial drugs or anti-viral drugs), apheresis treatments, ventilator support, fluid support or others.

In one embodiment, the treatment is selected from one or more of: antibiotic treatments (e.g. penicillins, cephalosporins, tetracyclines, aminoglycosides, macrolides, clindamycin, sulphonamides, trimethoprim, metronidazole, tinidazole, quinolones and/or nitrofurantoin), anti-microbial treatments (e.g. ethambutol, isoniazid, pyrazinamide, rifampicin, aminoglycosides (amikacin, kanamycin), polypeptides (capreomycin, viomycin, enviomycin), fluoroquinolones (ciprofloxacin, levofloxacin, moxifloxacin), thioamides (ethionamide, prothionamide), cycloserine (closerin), terizidone, rifabutin, macrolides (clarithromycin), linezolid, thioacetazone, thioridazine, arginine, vitamin D and/or R207910), anti-viral COVID treatments (e.g. remdesivir), anti-viral influenza treatments (e.g. amantadine, umifenovir, moroxydine, rimantadine, umifenovir, zanamivir and neuraminidase inhibitors, cap-dependent endonuclease inhibitors, adamantanes, peramivir, zanamivir, oseltamivir phosphate and baloxavir marboxil) as well as anti-viral treatments for other viral diseases that may lead to a high level of NETosis and anti-fungal treatments (e.g. clotrimazole, econazole, miconazole, terbinafine, fluconazole, ketoconazole and amphotericin).

In one embodiment, the treatment is an anti-inflammatory drug. Many steroidal and non-steroidal anti-inflammatory drugs are known in the art. Some examples of steroidal anti-inflammatory drugs include without limitation, dexamethasone, hydrocortisone, cortisone, betamethasone, prednisone, prednisolone, triamcinolone and methylprednisolone. Some examples of non-steroidal anti-inflammatory drugs include without limitation, aspirin, celecoxib, diclofenac, diflunisal, etodolac, ibuprofen, indomethacin, CD24Fc (CD24 protein attached to the Fc region of immunoglobulin G) and EXO-CD24 (CD24-Exosomes).

In one embodiment, treatment is a DNase treatment to digest excess NETs or an inhibitor of NETosis, such as an anthracycline drug. In a further embodiment, the anthracycline drug is selected from: epirubicin, daunorubicin, doxorubicin and idarubicin.

In one embodiment the treatment is a therapeutic antibody drug directed to bind to NETs or to a component part of a NET including, without limitation, a therapeutic antibody directed to bind to a nucleosome, or to any component part of a nucleosome. Examples include therapeutic antibodies directed to bind to nucleosomes containing histone isoform H3.1, citrullinated histones, myeloperoxidase, neutrophil elastase or C-Reactive Protein.

In one embodiment the treatment is a nucleic acid scavenger that adsorbs and/or removes nucleic acids from the circulation or from the body, for example the DNA scavenger polyamidoamine.

No treatments to inhibit NETosis have been used or, to the knowledge of the authors, tested for use in humans to date. There are a number of reasons for this. Firstly, as noted above, NETosis is an important component of the immune system. This will be particularly important in patients who may not yet have produced an antibody response to the infective agent and in whom the NETosis process, together with phagocytosis, may be the primary mode of fighting infection and preventing its spread. Moreover, in the case of NETosis inhibitor therapies, this is even more important because, rather than removing the products of NETosis (which may be replaced), the immune process itself is disabled preventing further production of NETs. Secondly, the half-life of DNase administered intravenously is reported to be 3-4 hours, but the half-life of anthracyclines is more than 40 hours and the anthracyclines persist in the circulation for at least several days. Disabling the immune system for several days in a patient with a severe infection would be sufficient time for a significant worsening of the disease. Thirdly, anthracyclines are cytotoxic drugs usually used to treat cancer by killing cancer cells. It is clear that an agent which disables one of the important modes of action of the immune system and is also long-lived and cytotoxic, should not be administered to a sick subject whose disease is not NETosis related and who does not require such treatment. Treatment of patients with normal or low NETs with a drug that inhibits NETosis is therefore inappropriate and potentially dangerous. Administration of such therapies therefore requires careful selection of patients with high NETs levels in need of treatment using the methods described herein.

Therefore, in one aspect of the invention there is provided a combination product comprising a drug for the treatment of a NETosis related disease and a companion diagnostic test for the detection or measurement of cell free nucleosomes. In some embodiments the combination product may comprise more than one drug (for example a NETosis inhibitor and a cardioprotective agent and/or an antibiotic) and/or more than one companion test (for example a test for cell free nucleosomes and a test for a cytokine moiety). In one embodiment the combination product comprises a DNase drug and a companion diagnostic test for the detection or measurement of cell free nucleosomes.

In one embodiment the combination product comprises a drug that digests NETs, for example a DNase drug that digests NETs composed of longer chains of nucleosomes into smaller fragments comprising shorter chains of oligonucleosomes or mononucleosomes and a companion diagnostic test for the detection or measurement of cell free nucleosomes.

In one embodiment the combination product comprises a nucleic acid scavenger and a companion diagnostic test for the detection or measurement of cell free nucleosomes.

In one embodiment the combination product comprises a therapeutic antibody drug directed to bind to NETs, a component part of a NET, MPO, NE or CRP and a companion diagnostic test for the detection or measurement of cell free nucleosomes.

In one embodiment, the combination product comprises a drug therapy that inhibits or prevents NETs formation by neutrophil cells and a companion diagnostic test for the detection or measurement of cell free nucleosomes. No such drugs that inhibit or prevent NETs formation are currently used to treat NETosis related diseases. It was reported by Figueiredo et al., Immunity (2013) 39: 874-884 that anthracycline drugs were effective in the treatment of sepsis induced in mice. A lethal dose of sepsis was induced in mice by cecal ligation and puncture that resulted in death in 100% of mice within 72 hours. Treatment with the anthracycline drug epirubicin resulted in 75% survival and a reduction in inflammation as measured by a variety of circulating inflammatory biomarkers. However, the mechanism of action of anthracyclines in achieving this effect could not be determined and Figueiredo was silent with respect to NETs or NETosis. Khan et al., Cancers (2019) 11: 1328 later investigated the effect of anthracycline drugs in vitro on neutrophil cells in cell culture and reported that they inhibit NETosis. Khan concluded that anthracyclines and other DNA chelating drugs could be considered as potential therapeutic drugs for suppressing unwanted NETosis in NETs-related disease. However, as the main toxic side effect of anthracycline drugs is cardiotoxicity, Khan proposed that anthracycline drugs could be administered in combination with dexrazoxane, a cardioprotective agent used for limiting the side effects of anthracyclines, that does not affect NETosis or the ability of anthracyclines to suppress NETosis.

Anthracycline drugs are highly effective chemotoxic agents for the treatment of cancer. However, they have a number of side effects including alopecia, skin rash, nausea and vomiting, malaise, fever, peripheral neurotoxicity, secondary leukaemia and cardiotoxicity. Myocardial toxicity is dose limiting because it may lead to potentially fatal congestive heart failure (CHF) during therapy or months to years after therapy. For example, the probability of developing impaired myocardial function based on a combined index of signs, symptoms and decline in left ventricular ejection fraction is estimated to be 1 to 2% at a total cumulative dose of 300 mg/m² of doxorubicin over repeated treatment cycles, 3 to 5% at a dose of 400 mg/m², 5 to 8% at 450 mg/m² and 6 to 20% at 500 mg/m². The risk of developing CHF increases rapidly with increasing total cumulative doses of doxorubicin in excess of 400 mg/m². A typical dose used for chemotherapy is 60-75 mg/m² per treatment cycle.

The dose of anthracyclines observed to be useful in the treatment of a mouse model of sepsis was 0.6 µg/g (Figueiredo et al.) which the inventors have determined is approximately equivalent to 25 mg/m² in an 80 kg human male. Thus, a single intra-venous dose is less than 1/10 of the dose that causes 1 to 2% myocardial toxicity. The concentration of anthracycline drug required for near complete inhibition of NETosis of neutrophil cells *in vitro* was 5 µM (Khan et al.) which is approximately equivalent to 3 µg/ml. Investigation of the pharmacokinetic properties of doxorubicin shows that a single 25 mg/m² bolus intravenous dose yields a serum concentration of 10 µg/ml serum. Moreover, the half-life of doxorubicin in the circulation is approximately 41 hours and the serum level remains above 3 µg/ml for around 4 days. Thus, a single 25 mg/m² bolus dose inhibits NETosis for several days and may be used as a treatment for sepsis or other NETosis related diseases. Single bolus dose levels, or repeated cumulative dose levels, below 25 mg/m² may also be effective.

A nanoparticle anthracycline drug formulation has been reported to provide a targeted NETosis inhibitor approach where active drug is released only in activated neutrophils and so avoids toxic side effects whilst also maintaining the NETosis capacity of remaining neutrophils to respond to further infection (Zhang et al., Science Advances 2019;5: eaax7964). Therefore, in one embodiment there is provided a combination product comprising a nanoparticle anthracycline drug formulation for the treatment of a NETosis related disease and a companion diagnostic test for the detection or measurement of cell free nucleosomes.

A therapy involving the removal of NETs and NETs degradation products from the circulation by plasmapheresis that provides a NETosis treatment that avoids pharmaceutical toxic side effects has been reported (WO2019053243). Therefore, in one embodiment there is provided a combination product comprising a plasmapheresis therapy for the treatment of a NETosis related disease and a companion diagnostic test for the detection or measurement of cell free nucleosomes.

Anti-inflammatory drug treatments for subjects suffering from COVID-19 based on CD24 protein have been reported. One example is CD24-Exosomes which has been reported to cure 29 of 30 moderate/serious COVID cases within several days (Times of Israel 5 Feb 2021 and ClinicalTrials.gov Identifier: NCT04747574) and involves delivery of CD24 in exosomes. Another such treatment, CD24Fc, comprises the nonpolymorphic regions of CD24 attached to the Fragment crystallizable region (Fc) region of a human IgG1 (ClinicalTrials.gov Identifier: NCT04317040). Therefore, in one embodiment there is provided a combination product comprising a plasmapheresis therapy for the treatment of a NETosis related disease and a companion diagnostic test for the detection or measurement of cell free nucleosomes.

NETs or NETosis related diseases include infectious diseases such as sepsis, pneumonia, COVID and influenza as well as other diseases involving a pathological elevation in NETs production including, without limitation, pneumonia, SARS or ARDS of any cause, thrombotic or micro-thrombotic conditions, many inflammatory disease conditions and NETosis related complications of other diseases including amputation and thrombotic complications of diabetes and thrombotic complications of cancer and many other diseases.

The methods may comprise:
(i) measuring the level of cell free nucleosomes (optionally in combination with the level of one or more interleukins) in a sample obtained from the subject;
(ii) identifying the subject as suffering from a NETosis related disease (such as an infection) in need of treatment based on a higher level of cell free nucleosomes compared to a control; and
(iii) administering a treatment to the subject.

In preferred embodiments the treatment is a DNase treatment to digest excess NETs, an anti-nucleosome or anti-NETs therapeutic antibody treatment, an apheresis or plasmapheresis treatment to remove excess NETs or an inhibitor of NETosis as described herein.

In one embodiment, there is a provided a method to identify a subject suffering from an infection who has, or is at risk of developing, a medical complication that requires treatment comprising the steps of:
(i) measuring the level of cell free nucleosomes (optionally in combination with the level of one or more interleukins) in a sample obtained from the subject;
(ii) identifying the subject as suffering from an infection in need of treatment based on a higher level of cell free nucleosomes compared to a control; and
(iii) administering a treatment to the subject.

In one embodiment the infection is sepsis or sceptic shock.

In a preferred embodiment, there is provided a method to identify a subject infected by a respiratory virus who has, or is at risk of developing, a medical complication that requires treatment comprising the steps of:
(i) measuring the level of cell free nucleosomes (optionally in combination with the level of one or more interleukins) in a sample obtained from the subject;
(ii) identifying the subject as at risk of developing a medical complication based on a higher level of cell free nucleosomes compared to a control; and
(iii) administering a treatment to the subject.

In preferred embodiments the respiratory infection is influenza or coronavirus and the medical complication is pneumonia. Suitable treatments may include, without limitation, respiratory support using extracorporeal oxygenation, respiratory support using a medical ventilator designed to provide mechanical ventilation of air into and out of the lungs of a patient who is physically unable to breathe sufficiently unaided and/or provision of oxygen and/or antiviral, antibacterial or anti-inflammatory drugs.

According to another aspect of the invention there is provided a method of treatment for an infection comprising identifying a patient in need of treatment for said infection using a panel test and providing said treatment, wherein the panel test comprises reagents to detect measurements of nucleosomes or components thereof. A patient with an infection is expected to have a higher level of cell free nucleosomes compared to a control.

### Therapeutic Antibodies

Therapeutic antibodies are administered intravenously to neutralise moieties that cause injury or disease in a subject. Therapeutic antibodies, and other similar or derived therapeutic binders such as Fab and Fv fragments, are normally human or humanized in the nature of their heavy and light chain amino acid sequences. Therapeutic antibodies and methods for their development and production are well known in the art. Therefore, in a further aspect of the invention there is provided an anti-nucleosome antibody for the treatment of severe hyperimmune reactions, including pneumonia.

Therefore, in a further embodiment, there is provided a method of treating a subject infected by a respiratory virus with a medical complication, comprising the steps of:
(i) measuring the level of cell free nucleosomes (optionally in combination with the level of one or more interleukins) in a sample obtained from the subject;
(ii) identifying the subject as having a medical complication based on a higher level of cell free nucleosomes compared to a control; and
(iii) administering a therapeutic anti-nucleosome antibody to the subject.

Prevention or inhibition of NETosis through treatment with NETosis inhibitors reduces the level of NETs and nucleosomes in the circulation and/or tissues of subjects suffering from conditions involving inappropriately high levels of NETs. This has been shown to lead to improved clinical outcomes for subjects suffering from NETosis related disease conditions such as sepsis or stroke (Figueiredo et al., Immunity (2013) 39: 874-884 and Zhang et al., Science Advances (2019) 5: eaax7964). Similarly, removal of NETs and nucleosomes from the circulation and/or tissues of a subject suffering from NETosis associated disease conditions leads to improved clinical outcomes.

The immunoassays described herein for the measurement of H3.1-nucleosomes, citrullinated nucleosomes, MPO and NE use high avidity and specificity monoclonal antibodies for binding to nucleosomes and NETs. These antibodies bind strongly and specifically to NETs, NETs metabolites and nucleosomes. These antibodies may therefore be used as therapeutic antibodies to bind to NETs *in vivo* to neutralise NETs and facilitate their clearance from the body, for example by phagocytosis (Weiskopf and Weissman, Mabs (2015) 7:303-10).

CRP is an acute phase protein known to be physically associated with NETs that can additionally induce NETosis. Antibodies to CRP may therefore neutralise and clear NETs and also inhibit the induction of NETosis through neutralisation and clearance of CRP.

Therefore, in one aspect of the invention there is provided a method of treating a NETosis related disease comprising the administration of a therapeutic antibody directed to bind to a nucleosome or component thereof, DNA, myeloperoxidase, neutrophil elastase or C-reactive protein. The therapeutic antibody is able to neutralise or promote the clearance of NETs from a subject suffering from the disease. Methods for the administration of therapeutic antibodies are well known in the art.

In another aspect of the invention there is provided an anti-nucleosome, anti-DNA, anti-myeloperoxidase, anti-neutrophil elastase or anti-C-Reactive Protein therapeutic antibody for use in the treatment of a condition involving excess or inappropriate NETosis (i.e. a NETosis related disease).

The anti-histone H3.1 antibody, anti-nucleosome antibody and anti-citrullinated H3 antibody used by the inventors for the assay of nucleosomes described herein, have been selected as highly avid and specific antibodies and are therefore particularly useful as therapeutic antibodies.

In particular, the anti-histone H3.1 antibody may be highly specific. Nucleosomes are subject to clipping in which the histone tail is physically and irreversibly removed by regulated proteolysis, or clipping. Furthermore, histone degradation has been shown to be involved in the formation of NETs (see Papayannopoulos et al. (2010) J. Cell Biol. 191(3): 677-691). On histone H3, clipping is reported to occur around amino acid position 21 (Yi and Kim (2018) BMB Reports, 51(5): 211-218). The amino acid sequence of histone H3.1 at positions 27-36 is KSAPATGGVK (SEQ ID NO: 1). The amino acid sequence at positions 29-35 does not include any commonly post-translationally modified amino acids (for example lysine, serine or arginine). Therefore, antibodies directed to bind to this epitope (i.e. amino acid positions 29-35) are unaffected, or minimally affected, by the post-translational modification status of the nucleosome, and will bind to all or most nucleosomes containing histone H3.1, regardless of PTM structure. Therefore, the solid phase capture antibody selected for use by the inventors for the immunoassay described herein, was an anti-histone H3.1 antibody directed to bind to an epitope located within the core of the histone near to amino acid position 30-33 so that both intact and clipped nucleosomes are captured by the antibody regardless of their PTM status. This maximises the capture of H3.1-nucleosomes and the efficacy of this approach is clear in Figure 6. The amino acid sequence of histone H3.1 is known in the art and is described at UniProt Accession No. P68431.

Therefore, in one embodiment of the invention, the therapeutic antibody is directed to bind to a core histone epitope of histone H3.1 at an amino acid epitope located higher than amino acid position 21. In a preferred embodiment, the anti-histone H3.1 therapeutic antibody is directed to bind to an epitope located within the core of the histone H3, at or near to amino acid position 29-35, in particular at or near to amino acid position 30-33.

The labelled antibody used by the inventors herein for immunoassay was an anti-nucleosome antibody directed to bind to a conformational nucleosome epitope present in intact nucleosomes containing a histone octamer core complexed with DNA. The antibody does not bind (or binds weakly) to free histone octamer complexes, free histones (i.e. without DNA), free DNA or free histones. Again, the antibody may be relatively unaffected by the histone PTM composition of the nucleosomes to be bound.

Therefore, in one embodiment of the invention the therapeutic antibody is directed to bind to a conformational nucleosome epitope present in intact nucleosomes containing a histone octamer core complexed with DNA.

In one embodiment of the invention the therapeutic antibody is directed to bind selectively to clipped nucleosomes, for example by binding to an epitope present in clipped nucleosomes wherein one or more histone tails have been removed. In this embodiment, the epitope may previously have been masked in intact nucleosomes by the presence of complete histone tails (thus preventing antibody binding). Therefore, the epitope bound by the antibody which is selective for clipped nucleosomes, may be an epitope that is not accessible in intact (i.e. whole or unclipped) nucleosomes. In one embodiment, the clipped nucleosome comprises a histone H3, H2A and/or H4 protein where the histone tail has been removed.

In one embodiment a mixture of 2 or more therapeutic antibodies may be administered to a subject. For example, without limitation, a mixture of one antibody directed to bind to a nucleosome epitope present in intact nucleosomes, together with another antibody directed to bind to a core histone epitope of histone H3.1.

The antibodies used by the inventors for immunoassay are mouse monoclonal antibodies. These mouse monoclonal antibodies would be useful therapeutic antibodies in mice. Use in humans or other animals will lead to an antigenic immune response and the generation of antimouse immunoglobulin antibodies (because the monoclonal antibodies are foreign proteins). To avoid an antigenic response, non-human monoclonal antibodies may be humanized. Humanized antibodies are non-human antibodies whose protein composition has been modified to be similar to that of naturally occurring human antibodies and are well known in the art. Antibodies consist of variable domains including complementarity-determining regions (CDRs) which are unique to the antibody and determine the antibody's epitope binding specificity and avidity, and constant domains which are species specific. One method of humanization involves the fusing of DNA coding for variable CDRs of a non-human antibody with DNA coding for human constant domains. This method can be used to produce a DNA vector coding for a largely human antibody that is not antigenic in human subjects and has the binding specificity and avidity of the original non-human monoclonal antibody. Humanized antibodies may be manufactured on a large scale using the resulting DNA vectors.

Therefore, in one embodiment the therapeutic antibody is a humanized antibody.

It will be understood that the embodiments described herein may be applied to all aspects of the invention, *i.e.* the embodiment described for the uses may equally apply to the claimed methods and so forth.

### EMBODIMENTS

1. A method of monitoring the progress of a disease in a subject suffering from an infection, comprising:
   (i) contacting a body fluid sample obtained from the subject with a binding agent to detect or measure the level of cell free nucleosomes or a component thereof;
   (ii) repeating step (i) on one or more occasions; and
   (iii) using any changes in the level of cell free nucleosomes or component thereof to monitor the progression of the infection in the subject.
2. A method of assigning a risk of an adverse outcome to a subject suffering from an infection, comprising:
   (i) contacting a body fluid sample obtained from the subject with a binding agent to detect or measure the level of cell free nucleosomes or a component thereof; and
   (ii) using the level of cell free nucleosomes detected to assign the likelihood of an adverse outcome to said subject,
   wherein a subject identified with a high likelihood of an adverse outcome is assigned for medical intervention.
3. The method as defined in embodiment 1 or embodiment 2, wherein the infection is a viral, bacterial, fungal or microbial infection.
4. The method as defined in any one of embodiments 1 to 3, wherein the infection is a respiratory tract infection.
5. The method as defined in embodiment 4, wherein the respiratory tract infection is selected from: influenza, pneumonia and severe acute respiratory syndrome (SARS).
6. The method as defined in embodiment 1 or embodiment 2, wherein the subject is suffering from sepsis or septic shock.
7. The method as defined in any one of embodiments 1 to 6, wherein the body fluid sample is a blood, serum or plasma sample.
8. The method as defined in any one of embodiments 1 to 7, wherein the cell free nucleosome is a part of, or derived from, a neutrophil extracellular trap.
9. The method as defined in any one of embodiments 1 to 8, wherein the component of the cell free nucleosome comprises an epigenetic feature of the cell free nucleosome.
10. The method as defined in embodiment 9, wherein the epigenetic feature is a histone isoform, such as a histone isoform of a core nucleosome, in particular a histone H3 isoform.
11. The method as defined in embodiment 10, wherein the histone isoform is H3.1.
12. The method as defined in embodiment 9, wherein the epigenetic feature is a histone post translational modification (PTM), such as a histone PTM of a core nucleosome, in particular a histone H3 or H4 PTM.
13. The method as defined in embodiment 12, wherein the histone PTM is selected from citrullination or ribosylation.
14. The method as defined in any one of embodiments 1 to 13, wherein the level of cell free nucleosomes or component thereof is detected or measured using an immunoassay, immunochemical, mass spectroscopy, chromatographic, chromatin immunoprecipitation or biosensor method.
15. The method as defined in any one of embodiments 1 to 14, wherein the method of detection or measurement comprises contacting the body fluid sample with a solid phase comprising a binding agent that detects cell free nucleosomes or a component thereof, and detecting binding to said binding agent.
16. The method as defined in any one of embodiments 1 to 15, wherein the method of detection or measurement comprises: (i) contacting the sample with a first binding agent which binds to an epigenetic feature of a cell free nucleosome; (ii) contacting the sample bound by the first binding agent in step (i) with a second binding agent which binds to cell free nucleosomes; and (iii) detecting or quantifying the binding of the second binding agent in the sample.
17. The method as defined in any one of embodiments 1 to 16, wherein the subject is a human or an animal subject.
18. The method as defined in any one of embodiments 1 to 17, additionally comprising comparing the level of cell free nucleosomes or component thereof in the body fluid sample of the subject with one or more controls.
19. The method as defined in embodiment 18, wherein the control is a healthy subject.
20. The method as defined in embodiment 18, wherein the control is a subject with the infection displaying no, or mild, symptoms.
21. The method as defined in any one of embodiments 1 to 20, wherein the level of cell free nucleosomes or component thereof is elevated compared to the control.
22. The method as defined in any one of embodiments 1 to 21, wherein the level of cell free nucleosomes is detected or measured as one of a panel of measurements.
23. The method as defined in embodiment 22, wherein the panel comprises one or more interleukins.
24. The method as defined in embodiment 23, wherein the one or more interleukins are selected from the group consisting of: IL-6 and IL-12.
25. The method as defined in any one of embodiments 22 to 24, wherein the panel comprises C reactive protein (CRP), myeloperoxidase (MPO), D-Dimer and/or factor VII-activating protease (FSAP).
26. The method as defined in any one of embodiments 22 to 25, wherein the panel comprises MPO.
27. A method of detecting a subject in need of medical treatment for pneumonia, acute respiratory syndrome (ARS), acute respiratory distress syndrome (ARDS) or severe acute respiratory syndrome (SARS), comprising:
   (i) contacting a body fluid sample obtained from the subject with a binding agent to detect or measure the level of cell free nucleosomes or a component thereof; and
   (ii) using the level of cell free nucleosomes as an indicator that the subject is in need of medical treatment for pneumonia, ARS, ARDS or SARS.
28. A method of detecting a subject in need of medical treatment for sepsis or septic shock, comprising:
   (i) contacting a body fluid sample obtained from the subject with a binding agent to detect or measure the level of cell free nucleosomes or a component thereof; and
   (ii) using the level of cell free nucleosomes as an indicator that the subject is in need of medical treatment for sepsis or septic shock.
29. A method of treating a NETosis related disease comprising the administration of a therapeutic antibody directed to bind to a nucleosome or component thereof, myeloperoxidase, neutrophil elastase or C-reactive protein.
30. The method as defined in embodiment 29, wherein the NETosis related disease involves high levels of neutrophil extracellular traps.
31. The method as defined in embodiment 29 or embodiment 30, wherein the NETosis related disease is a viral or bacterial infection.
32. The method as defined in any one of embodiments 29 to 31, wherein the therapeutic antibody is directed to bind to an epitope present in intact nucleosomes.
33. The method as defined in any one of embodiments 29 to 31, wherein the therapeutic antibody is directed to bind to an epitope present in a clipped nucleosome.
34. The method as defined in any one of embodiments 29 to 33, wherein the therapeutic antibody is directed to bind to a component of the nucleosome which is histone H3.1 or a citrullinated histone.
35. The method as defined in any one of embodiments 29 to 34, wherein the therapeutic antibody is directed to bind to a histone H3.1 epitope located at amino acid position 30-33 in the amino acid sequence of histone H3.1.

The invention will now be illustrated with reference to the following non-limiting examples.

### EXAMPLE 1

We induced NETs formation in white cells in fresh healthy whole blood samples by addition of heparin and then demonstrated detection of the NETs material produced in plasma (Lelliott *et* al, International Immunology (2019) pii: dxz084). We collected whole blood samples from two healthy volunteers, in whom low levels of circulating NET material would be expected, in EDTA plasma blood collection tubes and in heparin plasma blood collection tubes. The two EDTA plasma blood collection tubes were centrifuged immediately to separate the cellular and plasma fractions to minimise contamination by large chromatin (including NET material) and the plasma was transferred to cryotubes and frozen. The two heparin plasma blood collection tubes were incubated at room temperature for 1 hour with gentle rotation of the tubes. The tubes were then centrifuged and the plasma was transferred to cryotubes and frozen.

The samples were assayed for nucleosomes containing histone isoform H3.1 (H3.1-nucleosomes) using an ELISA procedure in duplicate. In brief, 20µl of sample were added to a microtiter well containing magnetic particles precoated with an anti-histone H3.1 antibody. The sample was incubated and the magnetic particles were isolated and washed. An anti-nucleosome antibody directed to bind to a conformational nucleosome epitope conjugated to horse radish peroxidase was added to the magnetic particles. The particles were incubated and then isolated and washed. The bound anti-nucleosome antibody was measured using a coloured substrate reaction. The results are shown in Figure 1 and show that the NET material level was high in the heparin tubes but low in the EDTA tubes. This clearly shows that elevated levels of circulating NET material can be detected in a simple low cost immunoassay test.

### EXAMPLE 2

DNA was extracted from the two heparin and plasma samples described in EXAMPLE 1. and applied to a chip-based capillary electrophoresis instrument (Agilent Bioanalyzer) to analyse the DNA by fragment size. DNA fragments of approximately 150bp size corresponding to mononucleosomes have a retention time of approximately 60 seconds. As shown in Figure 2, the level of mononucleosome associated DNA observed was low (as expected for healthy volunteers). The DNA fragment size corresponding to NET material has a longer retention time of approximately 110 seconds. As shown in Figure 2 the level of NET material was low in EDTA plasma (as expected for healthy volunteers), but high in the heparin plasma tubes in which NET formation was stimulated by exposure to heparin. This result confirms that the elevated nucleosome levels observed in heparin plasma in EXAMPLE 1. above were NET derived and not mononucleosomes.

### EXAMPLE 3

EDTA plasma samples are collected from 100 subjects who all tested positive for coronavirus infection, including 50 control subjects with mild symptoms and 50 test subjects who have respiratory complications and are in need for ventilator support. The circulating NET material is measured using a nucleosome immunoassay method as described in EXAMPLE 1. The 50 control subjects are found to have low NET nucleosome levels and these levels are used to establish a control range. The 50 test subjects are found to have higher NET nucleosome levels and these high levels are used as an indicator that, in addition to the viral infection, the subjects have respiratory complications that require treatment.

### EXAMPLE 4

The experiment conducted in EXAMPLE 3 is repeated but the immunoassay performed measured citrullinated nucleosomes. The 50 control subjects are found to have low citrullinated nucleosome levels and these levels are used to establish a control range. The 50 test subjects are found to have higher citrullinated nucleosome levels and these high levels are used as an indicator that, in addition to the viral infection, the subjects have respiratory complications that require treatment.

### EXAMPLE 5

The experiment conducted in EXAMPLE 3 is repeated but the immunoassay performed measures myeloperoxidase-nucleosome adduct levels. The 50 control subjects are found to have low myeloperoxidase-nucleosome levels and these levels are used to establish a control range. The 50 test subjects are found to have higher myeloperoxidase-nucleosome levels and these high levels are used as an indicator that, in addition to the viral infection, the subjects have respiratory complications that require treatment.

### EXAMPLE 6

The experiment conducted in EXAMPLE 3 is repeated but the immunoassay performed measures neutrophil elastase-nucleosome adduct levels. The 50 control subjects are found to have low neutrophil elastase-nucleosome levels and these levels are used to establish a control range. The 50 test subjects are found to have higher neutrophil elastase-nucleosome levels and these high levels are used as an indicator that, in addition to the viral infection, the subjects have respiratory complications that require treatment.

### EXAMPLE 7

The experiment conducted in EXAMPLE 5 is repeated but, in addition, the levels of CRP and IL6 are also measured in the EDTA plasma samples or in serum samples from the same subjects. An algorithm is developed using Logistic Regression analysis of the results to maximise the clinical sensitivity and specificity for the identification of test subjects.

### EXAMPLE 8

EDTA plasma samples were collected from 50 healthy subjects in 2019 prior to the COVID-19 outbreak and from 50 subjects admitted to hospital for symptoms of suspected COVID-19 infection. Of the 50 subjects admitted for symptoms of COVID-19 infection, 34 were tested as positive for COVID-19 infection using a polymerase chain reaction (PCR) test and 16 were tested as negative for COVID-19 infection using the PCR test.

The symptomatic patients were selected to include both patients who experienced a severe form of the disease and a milder form of the disease. 40 subjects were hospitalized including 5 subjects hospitalized in ICU of whom 2 subjects received mechanical ventilation for 9 and 10 days respectively. Hospitalized subjects who tested PCR negative for COVID-19 were none-the-less treated for the symptoms of respiratory infection.

H3.1-nucleosome levels were measured in the plasma samples and the results are shown in Figure 3. All PCR positive COVID-19 patients had elevated levels of plasma H3.1-nucleosomes and on this basis 100% of PCR positive COVID-19 patients were discriminated from normal controls at a specificity of 94% (3 false positives among the 50 control subjects) and an AUC of 98.7%.

The PCR positive COVID-19 patients could be divided into 2 groups on the basis of their H3.1-nucleosome levels. The first group, comprising 15 of the 34 subjects, had H3.1-nucleosome levels below 600ng/ml. The second group, comprising 19 of the 34, subjects had H3.1-nucleosome levels that were higher than the upper range limit of the assay (>700ng/ml).

A similar pattern was observed for the 16 patients suffering with COVID-19 symptoms but tested negative by PCR of whom 6 had H3.1-nucleosome levels below 600ng/ml and 10 had H3.1-nucleosome levels >700ng/ml.

### EXAMPLE 9

In order to determine whether circulating H3.1-nucleosome levels are predictive of COVID-19 disease severity, EDTA plasma samples were collected from 14 subjects diagnosed with COVID-19 infection by means of a positive PCR COVID-19 viral test result. Of these, 5 samples were collected from subjects attending at an outpatient hospital appointment or at presentation at the hospital Emergency Room (ER), 3 samples were collected from patients hospitalized in normal dependency wards (i.e. not in an intensive care unit) and 6 samples were collected from patients with very severe disease who were transferred to the intensive care unit (ICU) of a tertiary hospital centre for high levels of respiratory and other clinical support including mechanical ventilation and Extracorporeal Membrane Oxygen (in which patients are cannulated and the function of the patient's lungs is replaced by pumping the blood through an artificial oxygenator). The mortality of these subjects was 4 deaths among the 6.

Circulating H3.1-nucleosome levels were measured in the plasma samples and the results are shown in Figure 4. There was a clear increase in levels between the subjects tested at outpatients/ER and those hospitalized for COVID-19 infection in regular wards. The Area Under the Curve (AUC) for this discrimination was 100%. There was also a clear increase in levels between the subjects hospitalized for COVID-19 infection in regular wards compared to those hospitalized in ICU. The AUC for this discrimination was also 100%. Moreover, the 4 patients who died were found to have the 4 highest circulating H3.1-nucleosome levels. The AUC for prediction of mortality was also 100%.

The results show that circulating H3.1-nucleosome levels are predictive of disease severity and mortality and may be used prognostically. The methods of the invention can therefore be used to predict the level of care required for a patient or subject at various stages of the clinical process including at presentation with respiratory infection or at later stages to determine the level of clinical support and/or the nature of the treatment required.

Similarly, the results show that circulating H3.1-nucleosome levels may be used to monitor patients by serial sampling to determine whether levels are rising or falling to inform clinical decisions on future treatment and clinical support regimes. For example, a falling level may inform a decision that intensive respiratory support is no longer essential and/or that the treatment used is effective.

Conversely a rising level may inform a decision that intensive respiratory support is required and/or that the current treatment used is not effective and that additional or alternative treatments should be considered. Therefore, the methods of the invention may be used to select patients in need of a treatment for a NETosis related condition including, for example and without limitation, a NETosis inhibitor drug, a treatment to remove NETs by apheresis or plasmapheresis or an anti-inflammatory treatment such as a CD24 treatment (e.g. EXO-CD24 or CD24Fc). Thus, the methods of the invention may be used as companion diagnostic products to drugs and treatments for diseases involving NETosis.

In a similar vein, this also indicates that the methods of the invention may be suitable for use for the assessment of the effectiveness of investigational drugs to treat respiratory disease conditions. In one embodiment circulating H3.1-nucleosome levels may be used as a surrogate endpoint in a drug trial either alone or in combination with other parameters.

### EXAMPLE 10

In order to determine if methods of the invention are effective for other nucleosome moieties, the same subjects described in EXAMPLE 9, were also tested for levels of circulating nucleosomes containing the histone modification of citrullination of the arginine residue at position 8 of histone H3 (H3R8Cit-nucleosome). This nucleosome moiety was selected because NETs chromatin is known to be citrullinated. The results are shown in Figure 5 and confirm that the methods of the invention are effective using citrullinated nucleosome measurements and more generally using measurements of any circulating nucleosome moiety or NETs moiety including MPO, NE or other NETs component moieties.

The results show that circulating H3R8Cit-nucleosome levels are predictive of disease severity and mortality and may be used prognostically. Similarly, H3R8Cit-nucleosome levels may be used to predict the level of care required for a patient or subject at various stages of the clinical process and to monitor patients by serial sampling as described above in EXAMPLE 9 for H3.1-nucleosome levels.

### EXAMPLE 11

A blood sample is taken from a subject suffering from a NETosis related disease and is assayed for nucleosomes as described herein as an indicator of excessive production or over production of NETs by the subject. If the level of NETs measured is below a threshold cut-off value the patient is not given a NETosis inhibiting therapy, such as an anthracycline therapy. If the level of NETs measured is above a threshold cut-off value the patient is given a NETosis inhibiting therapy, such as an anthracycline therapy. Further blood samples are taken at appropriate intervals (for example every 4 hours or daily) to monitor the fall in NETs levels in the subject's circulation to determine the effectiveness of the treatment. Used in this manner, the methods of the invention provide a combination product comprising a NETosis inhibitor therapy and a companion diagnostic for patient selection and monitoring of patients with pathological NETs levels in need of a NETosis therapy (e.g. an anti-viral or antibacterial drug, an anti-inflammatory drug, a blood thinning or clotting inhibitor drug, a NETosis inhibitor drug, a DNase drug, an anti-nucleosome therapeutic antibody drug, an anti-MPO therapeutic antibody drug, and anti-NE therapeutic antibody drug or an apheresis or plasmapheresis treatment to remove NETs from the circulation).

### EXAMPLE 12

Sepsis was induced in 16 pigs by infection with Escherichia coli (*E*. *coli*) bacteria administered by intravenous infusion over 3 hours (0-3 hours in Figures 6 and 7). The septic pigs were treated by a plasmapheresis method to remove NETs from the blood stream as described in WO2019053243. Briefly, whole blood was removed from the body of the pig through a tube into a plasmapheresis device, the whole blood was separated into a cell fraction and a plasma fraction, the plasma was passed through a plasmapheresis cartridge containing a binder of NETs to remove NETs from the plasma, the plasma was then re-joined with the blood cells and returned to the body of the pig. The plasmapheresis treatment was performed over 5 hours (2-7 hours in Figures 6 and 7). The plasmapheresis cartridges used for 9 of the pigs contained the NETs binder (treated pigs) and the cartridges used for the other 7 pigs contained no binder (control pigs).

Eight plasma samples were collected hourly at time points 0-7 hours post commencement of infection from each pig. Circulating nucleosomes were measured to ascertain whether the method of the invention was (i) effective as a monitor for the course of the infection and (ii) effective as a monitor for efficacy of the treatment.

In addition, plasma samples were collected from the plasmapheresis device, both upstream of the cartridge (to sample the plasma entering the NETs binder cartridge) and downstream of the cartridge (to sample the plasma leaving the NETs binder cartridge) to ascertain whether the extent of the depletion of the plasma by the NETs binder in the cartridge could be monitored by methods of the invention. Five upstream and five downstream samples were taken hourly at time points 3-7 hours post commencement of infection (3-7 hours in Figures 6 and 7).

The plasma samples were assayed for nucleosomes containing histone isoform H3.1 (H3.1-nucleosome) levels. Assay measurements for were performed by immunoassay using an automated immunoassay instrument. Briefly, calibrant or sample (50µl) was incubated with an acridinium ester labelled anti-nucleosome antibody (50µl) and assay buffer (100µl) for 1800 seconds at 37°C. Magnetic beads coated with an anti-histone H3.1 antibody (20µl) were added and the mixture was incubated a further 900 seconds. The magnetic beads were then isolated, washed 3 times and magnetic bound acridinium ester was determined by luminescence output over 7000 milliseconds.

Mean results for circulating H3.1-nucleosome levels in the control pigs and treated pigs are shown in Figure 6a. The control pigs (infected to induce sepsis but not treated) developed sepsis over the following hours and this was reflected in an observed rise in circulating H3.1-nucleosome levels. The rise in mean H3.1-nucleosome levels was clear at 1 hour (post commencement of infection) and accelerated after 3 hours which is consistent with the time course of the NETosis process. The H3.1-nucleosome levels continued to rise and reached 361ng/ml at 7 hours. A similar initial rise in mean circulating H3.1-nucleosome levels was observed in the treated pigs from 0-2 hours. Initiation of plasmapheresis treatment at 2 hours resulted in a slowing of the increase in nucleosome levels and the mean level observed at 7 hours was 150ng/ml. This is considerably lower than the mean level observed in the control pigs which demonstrates the effectiveness of the plasmapheresis method and shows that the level of H3.1 nucleosomes is an effective monitor and treatment guide for the course and extent of the sepsis disease and an effective monitor for the NETosis process *in vivo.*

Mean results for plasma H3.1-nucleosome levels measured in samples taken from within the plasmapheresis device upstream from the cartridge during operation are shown in Figure 6b. These results are similar to those observed for the mean circulating H3.1-nucleosome levels measured shown in Figure 6a.

Mean results for plasma H3.1-nucleosome levels measured in samples taken from within the plasmapheresis device downstream from the cartridge during operation are shown in Figure 6c. For the control pigs, the results of Figure 6c are similar to those in Figure 6b (and 6a) showing that passing plasma through a cartridge containing no binder of NETs did not significantly affect the observed H3.1-nucleosome level. This is consistent with the expected outcome that the level of NETs in plasma was not significantly affected by passage through a cartridge containing no binder of NETs. For the treated pigs, the results of Figure 6c are all low. This is consistent with the expected outcome that passing plasma through a cartridge containing a binder of NETs resulted in the removal of most or all NETs from the plasma. Moreover, the results show that NETs binder within the cartridge was not saturated with NETs at 7 hours and continued to bind all or most of the NETs present in plasma entering the device. Measurements of the level of H3.1-nucleosomes are therefore useful to determine when the binding material within a cartridge has become saturated and is hence no longer useful as a tool for the removal of NETs and should be exchanged for a fresh cartridge.

The combined results of Figures 6b and 6c therefore show that measurements of the level of H3.1-nucleosomes are useful as a monitor and a guide for treatments for NETosis and sepsis.

The results for circulating H3.1-nucleosome levels measured in samples taken from all 16 pigs are shown individually in Figure 7a. The mean H3.1-nucleosome level observed in control pigs at 7 hours was 361ng/ml and the level was above 120ng/ml in all control pigs (range 123-743ng/ml). In contrast, the mean H3.1-nucleosome level observed in treated pigs at 7 hours was 150ng/ml and the level was below 120ng/ml in most (7 of 9) treated pigs (range 27-111ng/ml). The results demonstrate the effectiveness of the plasmapheresis treatment method. The results also show that the level of H3.1 nucleosomes is an effective monitor and treatment guide for the course and extent of sepsis disease and an effective monitor and treatment guide for excessive NETosis *in vivo.* Moreover, the results in Figure 7a show that measurements of circulating H3.1-nucleosome levels can be used to identify individuals with elevated levels of NETs as suitable candidates for treatments to reduce levels of NETs or NETosis.

The 7 control pigs had elevated nucleosome levels and also had elevated indicators of clinical stress and required more intensive medical support than the 7 treated pigs with lower nucleosome levels. Moreover, the 2 treated pigs observed to have H3.1-nucleosome levels above 120ng/ml also had elevated indicators of clinical stress and required more intensive medical support. Therefore, the method of the invention is a successful method for the monitoring of the efficacy of NETosis treatments.

Results for plasma H3.1-nucleosome levels measured in samples taken from within the plasmapheresis device upstream from the cartridge during operation are shown individually for all 16 pigs in Figure 7b. As described above for Figure 6, the results shown in Figure 7b are similar to those in Figure 7a. The mean H3.1-nucleosome level observed in control pigs at 7 hours was 368ng/ml (range 121-629ng/ml). In contrast, the H3.1-nucleosome level observed in treated pigs at 7 hours was lower with a mean result of 143ng/ml (for all treated 9 pigs). For the 7 responder pigs the range of results at 7 hours was 34-127ng/ml.

Results for plasma H3.1-nucleosome levels measured in samples taken from within the plasmapheresis device downstream from the cartridge during operation are shown individually for all 16 pigs in Figure 7c. The mean level of H3.1-nucleosomes measured for control pigs at 7 hours was 378ng/ml (range 147-617ng/ml). In contrast, the mean level of H3.1-nucleosomes observed in plasma downstream of the cartridge at 7 hours for treated pigs was 2.4ng/ml (range 0.7-6.5ng/ml) and was below 7ng/ml at all time points for all 9 treated pigs.

The combined results of Figures 7b and 7c show that measurements of the level of H3.1-nucleosomes are useful as a monitor and a guide for treatments for NETosis and sepsis.

In combination, the results shown in Figure 7 indicate that the plasmapheresis treatment regime was successful in removing NETs from the circulation of all 9 treated pigs and that 7 of the 9 pigs responded well to that treatment but 2 were non-responders. These nucleosome results correlated extremely well with the clinical observations of the pigs.

Moreover, the 7 treatment responder pigs can be clearly differentiated from treatment non-responder pigs and untreated pigs on the basis of observed nucleosome results.

### EXAMPLE 13

Plasma samples were obtained from 20 human subjects diagnosed with sepsis and 10 healthy human subjects. The plasma samples were assayed for nucleosomes containing histone isoform H3.1 (H3.1-nucleosome) levels using an automated immunoassay instrument as described in EXAMPLE 12. Elevated levels were observed in sepsis samples compared to healthy subjects, which is likely due to the effect of NETosis at various stages of this disease (Figure 8).

## Claims

1. A method of monitoring the progress of a NETosis related disease in a subject, comprising:
(i) contacting a body fluid sample obtained from the subject with a binding agent to detect or measure the level of cell free nucleosomes comprising histone H3.1, wherein the level of cell free nucleosomes comprising H3.1 is detected or measured using an immunoassay method;
(ii) repeating step (i) on one or more occasions; and
(iii) using any changes in the level of cell free nucleosomes or component thereof to monitor the progression of the disease in the subject.

2. A method of assigning a risk of an adverse outcome to a subject, comprising:
(i) contacting a body fluid sample obtained from the subject with a binding agent to detect or measure the level of cell free nucleosomes comprising histone H3.1, wherein the level of cell free nucleosomes or component thereof is detected or measured using an immunoassay method; and
(ii) using the level of cell free nucleosomes comprising H3.1 detected to assign the likelihood of an adverse outcome to said subject,
wherein a subject identified with a high likelihood of an adverse outcome is assigned for medical intervention.

3. The method as defined in claim 1 or claim 2, wherein the subject is suffering from an infection, such as a viral, bacterial, fungal or microbial infection.

4. The method as defined in any one of claims 1 to 3, wherein the subject is suffering from a condition involving pathological clinical complications of high levels of NETs or NETosis.

5. The method as defined in claim 4, wherein the condition is selected from: pneumonia; COVID; influenza; severe acute respiratory syndrome (SARS); acute respiratory syndrome (ARDS); a thrombotic condition; a micro-thrombotic condition; an inflammatory disease condition; or a NETosis-related complication of amputation, a thrombotic complication of diabetes or a thrombotic complication of cancer.

6. The method of claim 2, wherein the adverse outcome is selected from: an acute coronary or cardiac event (such as a myocardial infarction or stroke); acute multi- or single-organ failure (such as renal failure, liver failure or heart failure); onset of a debilitating acute respiratory condition (such as pneumonia, hypoventilation/bradypnea, acute respiratory distress syndrome (ARDS) severe acute respiratory syndrome (SARS), bronchiolitis or bronchitis).

7. The method as defined in any one of claims 1 to 6, wherein the body fluid sample is a blood, serum or plasma sample.

8. The method as defined in any one of claims 1 to 7, wherein more than one epigenetic feature of cell free nucleosomes is measured as a combined biomarker, optionally wherein the epigenetic feature is a histone post translational modification (PTM), such as a histone PTM of a core nucleosome, in particular a histone H3 PTM.

9. The method as defined in any one of claims 1 to 8, wherein the method of detection or measurement comprises: (i) contacting the sample with a first binding agent which binds to an epigenetic feature of a cell free nucleosome, wherein the epigenetic feature is histone H3.1; (ii) contacting the sample bound by the first binding agent in step (i) with a second binding agent which binds to cell free nucleosomes; and (iii) detecting or quantifying the binding of the second binding agent in the sample.

10. The method as defined in claim 9, wherein the first binding agent is an anti-histone H3.1 antibody directed to bind to an epitope around amino acids 30-33 of the histone H3.1 protein and/or wherein the second binding agent is an anti-nucleosome antibody directed to bind to a conformational nucleosome epitope.

11. The method as defined in any one of claims 1 to 10, wherein the subject is a human or an animal subject.

12. The method as defined in any one of claims 1 to 11, additionally comprising comparing the level of cell free nucleosomes or component thereof in the body fluid sample of the subject with one or more controls, optionally wherein the control is a healthy subject, and/or wherein the level of cell free nucleosomes or component thereof is elevated compared to the control.

13. The method as defined in any one of claims 1 to 12, wherein the level of cell free nucleosomes is detected or measured as one of a panel of measurements, optionally wherein the panel comprises one or more interleukins, such as IL-6 or IL-12, C reactive protein (CRP), myeloperoxidase (MPO), D-Dimer and/or factor VII-activating protease (FSAP).

14. A combination product comprising a drug for the treatment of a NETosis related disease and a companion diagnostic test for the detection or measurement of cell free nucleosomes as defined in method claims 1 to 13.

15. The combination product as defined claim 14 comprising more than one drug (for example a NETosis inhibitor and a cardioprotective agent and/or an antibiotic) and/or more than one companion diagnostic test (for example a test for cell free nucleosomes and a test for a cytokine moiety), particularly the combination product comprises a DNase drug and a companion diagnostic test for the detection or measurement of cell free nucleosomes as defined in method claims 1 to 13.
